# EUROPÄISCHE PATENTSCHRIFT

(11) **EP 2 170 421 B1**
(45) Veröffentlichungstag und Bekanntmachung des Hinweises auf die Patenterteilung: **22.07.2015**
(21) Anmeldenummer: 08801121.8
(22) Anmeldetag: 01.08.2008
(51) Int. Cl.: A61L 29/14, A61L 29/16

(54) **VERBESSERTE ARZNEIMITTELBESCHICHTETE MEDIZINPRODUKTE UND DEREN HERSTELLUNG**
IMPROVED PHARMACEUTICAL-COATED MEDICAL PRODUCTS AND THE PRODUCTION THEREOF
PRODUITS MÉDICAUX AMÉLIORÉS AVEC REVÊTEMENT MÉDICAMENTEUX ET LEUR FABRICATION

(30) Priorität: 03.08.2007 DE 102007036685
(43) Veröffentlichungstag der Anmeldung: 07.04.2010
(60) Teilanmeldung: 13181006.1
(73) Patentinhaber: Invatec Technology Center GmbH, 8500 Frauenfeld (CH)
(72) Erfinder: SCHELLER, Bruno, 66121 Saarbrücken (DE); SPECK, Ulrich, 10098 Berlin (DE)
(74) Vertreter: Zimmermann & Partner Patentanwälte mbB
(86) Internationale Anmeldenummer: PCT/DE2008/001285
(87) Internationale Veröffentlichungsnummer: WO 2009/018816

(56) Entgegenhaltungen:
- WO-A1-95/03083
- WO-A1-2004/006976
- WO-A2-2004/028610
- WO-A2-2008/063576
- US-A1- 2002 165 521
- US-A1- 2005 059 965
- US-A1- 2007 003 596

## Beschreibung

### 1. Medizinischer und technischer Hintergrund

Zahlreiche Erkrankungen betreffen nicht den gesamten Organismus gleichzeitig sondern sind auf bestimmte Gewebearten, häufig auch auf sehr begrenzte einzelne Gewebebezirke oder Organteile beschränkt. Beispiele dafür finden sich unter den Tumor-, Gelenk- und Gefäßerkrankungen, insbesondere solide Tumore und die arterielle Gefäßerkrankung.
Die Pharmakotherapie auch dieser Erkrankungen erfolgt im allgemeinen durch orale oder intravenöse Gabe von Arzneistoffen, die sich im ganzen Körper verteilen und in vielen Fällen gerade bei schweren Erkrankungen unerwünschte Wirkungen in gesunden Geweben und Organen verursachen, die die therapeutische Anwendung begrenzen. Eine selektive Therapie der erkrankten Gewebe wurde mittels spezifisch an erkranktes Gewebe bindende Arzneistoffe (z. B. Antikörper) unter Beibehaltung des Applikationsweges oder durch selektive Verabreichung z.B. durch direkte Verabreichung in das erkrankte Gewebe oder durch Zufuhr über Katheter in die betroffenen Blutgefäße erreicht. Im Falle der selektiven Verabreichung entstehen durch die meist kurze Wirkdauer der Arzneistoffe und die invasiven Verabreichungswege Probleme, da sich eine beliebig wiederholte Gabe verbietet.

Probleme für die Pharmakotherapie ergeben sich durch den speziellen Verabreichungsweg und die Notwendigkeit, mit einer einmaligen Applikation einen wesentlichen prophylaktischen oder therapeutischen Effekt zu erreichen. In den vergangenen 10 Jahren sind bedeutende Erfolge insbesondere in der Behandlung arteriosklerotischer Gefäßveränderungen erzielt worden. Solche Veränderungen treten häufig lokal auf. Sie führen an bestimmten Gefäßabschnitten zu Verengungen oder Verschlüssen, die die Blutversorgung der nachgeschalteten Gewebe beeinträchtigen oder unterbinden. Betroffen sind vor allem das Herz, die Beine, das Hirn, die Nieren und chirurgisch veränderte Gefäße wie z. B. Dialyseshunts. Verengungen dieser Gefäße können durch perkutan eingeführte Katheter behandelt werden, die sich wegen ihres geringen Durchmessers ohne große Verletzung in die betreffenden Blutgefäße einführen lassen. Sie enthalten im distalen Teil meist einen in Falten um den Katheterschaft gerollten, mit Flüssigkeit aufdehnbaren Ballon. Dieser Ballon wird in gefaltetem Zustand in die verengte Stelle des Blutgefäßes vorgeschoben und dort für kurze Zeit (Sekunden bis wenige Minuten) aufgedehnt, so dass das ursprüngliche Gefäßlumen wieder hergestellt wird und das Blut den ursprünglich verengten Bereich wieder passieren kann. Zur Abstützung des eröffneten Gefäßlumens kann gleichzeitig ein röhrenförmiges Metallgeflecht (Gefäßstütze, Stent) eingebracht werden, das entweder auf dem gefalteten Ballon montiert ist oder als elastischer, selbst-expandierender Stent mit einem besonderen Katheter freigesetzt wird.

Während die initiale Erfolgsrate gemessen an einer Erweiterung des Gefäßlumens auf annähernd das Maß vor Einsetzen der Verengung bei über 90% liegt kommt es bei vielen Patienten wenige Monate nach der Behandlung zu einer erneuten Verengung (Restenose). Die wesentlichste Ursache ist eine durch die Verletzung bei der gewaltsamen Aufdehnung ausgelöste übermäßige Proliferation der Gefäßwand, insbesondere der glatten Muskelzellen, die auch nach Abheilen der ursprünglichen Verletzung nicht zum Stillstand kommt. Dieser Prozeß konnte in den Koronarien durch Beschichtung der Stents mit antiproliferativ wirksamen Arzneimitteln nahezu vollständig unterdrückt werden. Voraussetzung ist, dass die Arzneimittel langsam, d.h. über Tage und Wochen aus einer Polymermatrix freigesetzt werden. Nachteil der Beschichtung von Stents ist die Hemmung der Einheilung. An den Streben der Stents können sich Thromben bilden solange diese direkten Kontakt zum Blut haben. Thromben können zum plötzlichen und totalen Gefäßverschluß, Infarkt und Tod führen. Die Streben müssen daher rasch und dauerhaft von einer Endothelschicht überwachsen werden. Dies wird durch eine anhaltende Freisetzung eines die Zellvermehrung hemmenden Wirkstoffs verhindert.

Für periphere Arterien liegen keine kontrollierten Studien vor, die eine wirksame Prophylaxe der Restenose durch Beschichtung von Stents mit Arzneimitteln zeigen. Allerdings scheinen bestimmte selbstexpandierende Nitinol-Stents die Restenoserate etwas zu vermindern, ohne dass dafür eine Beschichtung mit Arzneimitteln notwendig ist (Schillinger M, Sabeti S, Loewe C et al. Balloon angioplasty versus implantation of nitinol stents in the superficial femoral artery. J N Engl J Med 2006; 354: 1879-88)

Im EP 1 372 737 A wird die Beschichtung von Ballonen im Prinzip beschrieben. Der Wirkstoff wird beispielsweise durch Tauchen des Ballons in eine wirkstoffhaltige Lösung aufgebracht. In der WO 2004 / 028582 A werden Möglichkeiten zur Beschichtung von Ballonen in unterschiedlichen, auch vorgeformten Faltungsstadien beschrieben.

Verengte Arterien, oft verbunden mit massiver Verkalkung, lassen sich meist nur durch hohen Druck (8-20 atm) wieder auf ihr ursprüngliches Lumen aufweiten. Dies wird durch druckresistente Ballone erreicht, deren Durchmesser sich mit steigendem Innendruck nicht wesentlich ändert. Die Ballone formen unter Druck einen starren Zylinder, der der Gefäßwand eng anliegt sofern der Durchmesser des Lumens des Gefäßes vor Expansion des Ballons kleiner war als der Durchmesser des Ballons. Ein außen aufgetragener Wirkstoff wird mit entsprechend hohem Druck an die aufgedehnte Gefäßwand gepresst.

Eine lokale Arzneimitteltherapie kann auch erforderlich sein, ohne dass Aufdehnung des Gefäßlumens erfolgen soll. Beispiele sind die Behandlung von Arterien nach Entfernung von Plaque-Material mit mechanischen (z.B. Atherectomie-Kathetern) oder thermischen Verfahren (z.B. Laser) oder auch die Behandlung von Gefäßwandveränderungen, die nicht zu flußbehindernden Stenosen führen (z.B. vulnerable Plaques, aufgelagerte Thromben). Eine Überdehnung und Gefäßverletzung ist in solchen Situationen unerwünscht. Werden die gebräuchlichen Angioplastie-Ballone in einem Durchmesser gewählt, der nicht zu einer Dehnung des Gefäßes führt, liegt deren Membran nur stellenweise der unregelmäßig geformten Gefäßwand an und überträgt nur dort den Arzneistoff.

### 2. Stand der Technik

Mit der WO 02/076509 A wurde erstmals offenbart, dass eine wenige Sekunden anhaltende Exposition der geschädigten Gefäßwand ausreicht, eine sich über Wochen entwickelnde Restenose zu verhindern. Desgleichen wurden mit Arzneimittel beschichtete Ballonkatheter beschrieben, die den Wirkstoff bei Kontakt mit der Gefäßwand in sofort bioverfügbarer Form freigeben.

In mehreren früheren und späteren Patentanmeldungen wurde die Beschichtung von Ballonkathetern mit Arzneimitteln beschrieben, wobei jedoch stets versucht wurde trotz der kurzen Kontaktzeit der Angioplastieballone mit der Gefäßwand einen anhaltenden Wirkstoffspiegel durch verzögerte Freisetzung zu erzielen. Die Methoden der Beschichtung führen soweit überhaupt beschrieben zu Produkten, die wesentliche Qualitätsmängel aufweisen und/oder nur aufwändig und teuer herzustellen sind.

Lipophile, wenig wasserlösliche Wirkstoffe wurden gegenüber hydrophilen Wirkstoffen bevorzugt, da lipophile Substanzen mit organischen, leicht flüchtigen Lösungsmitteln gut aufzutragen sind, bei der Handhabung und im Blut nicht so leicht vorzeitig von den Ballonoberflächen abgewaschen werden, rascher in Zellen aufgenommen werden und dort länger verweilen. In Einzelfällen haben hydrophile Wirkstoffe wie Methotrexat oder Arsentrioxid auf Stents Anwendung zur Hemmung einer Restenose durch Neointimahyperplasie gefunden (US 20060348947; Yang W, Ge J, Liu H et al. Cardiovascular Research 2006;72:483-493). Die Wirkstoffe werden in wasserunlösliche Polymere eingeschlossen aus denen sie nur langsam freigesetzt werden. Auf diese Weise wird ein vorzeitiger Verlust an Wirkstoff verhindert. Das Gleiche gilt für die Verwendung hydrophiler Zytostatika zur antimikrobiellen Beschichtung von Dauer-Kathetern und anderen Implantaten (WO03099346).

Tatsächlich haben nur die in der WO 02/076509 A und WO 2004/028582 A offenbarten Katheterbeschichtungen zu wirksamen Produkten geführt, die Ausmaß und Häufigkeit der Restenose nach Gefäßdilatation beim Patienten vermindern (Scheller B, Hehrlein C, Bocksch W, Rutsch W, Haghi D, Dietz U, Böhm M, Speck U. Treatment of Coronary In-stent Restenosis with a Paclitaxel-coated Balloon Catheter. N Engl J Med 2006; 255: 2113-2124, Tepe G, Zeller T, Albrecht T, HellerS, Schwarzwälder U, Beregi J-P, Claussen CD, Oldenburg A, Scheller B, Speck U. Local delivery of paclitaxel to inhibit restenosis during angioplasty of the leg. New Engl J Med 2008;358:689-699).
In den Dokumenten des Standes der Technik werden zahlreiche Wirkstoffe und Matrix-Substanzen genannt, mit denen beschichtet werden kann: Bevorzugt genannte Wirkstoffe sind solche, die die Zellproliferation hemmen, entzündungs- und gerinnungshemmend wirken.
An Hilfststoffen wurden genannt: Kontrastmittel, matrix- oder gelbildende Hilfstoffe, z.B. Lipide oder in der Pharmazie gebräuchliche Polymere, Heparin, Rhizinusöl (WO 02/076509) oder Matrixsubstanzen bis 5000 D, hydrophil, Farbstoffe wie z.B. Indocyaningrün, Fluorescein, Methylenblau, Zucker, Zuckerderivate, niedermolekulares PEG, organische und anorganische Salze, Benzoate, Salicylate (WO 2004/028582) Polymere, auch zur Ummantelung von Arzneistoffen (EP 0519063; US 5,102,402).
Polymere wie z.B. Stärke, Gelatine, PEG, Albumin, Chitosan, ß-Cyclodextrine, Hydroxyethylcellulose sowie Lipide, die amphiphilen Phospholipide und Röntgenkontrastmittel einschließlich der amphiphilen lodoxaminsäure (DE 102004046244), Substanzen, die die Zellpermeabilität erhöhen wie Linolsäure, Linolensäure, Ölsäure, Stearinsäure, Phenylsalicylat; Antoxidantien wie Vitamin E, Tocotrienole, Tocopherole, sowie Nitrophenyloctylether, Bisethylhexylsebacat, Diisododecylphthalat, N-Methylpyrrolidon, Butylhydroxyanisol, Butylhydroxtoluen, Phosphorylcholin und Polymere (WO 2004/022124); Öle, Fettsäuren, Fettsäureester, Kontrastmittelderivate, Aminosäuren, Peptide, Vitamine, o-Phosphoserin, neutrale oder geladene amphiphile Substanzen, Salze (WO 2007090385); amphiphile Substanzen wie Polyethylenglycolester , Fettsäureester von Zuckern, Polyglyceryl-6-Fettsäureester, Polyglyceryl-10-Fettsäureester, Sucrosemonopalmitate, oberflächenaktive Stoffe mit Lipidketten, die sich in Lipidmembranen eingliedern, ionische und nichtionische Detergentien, Substanzen mit mehr als 4 Hydroxyl-, Carboxyl- oder Amino-Gruppen, Sorbitanfettsäureester, Substanzen mit einem Phenolring, Natriumcholat, Natriumtaurocholat; weiterhin Vitamine und Derivative, Polyethylenglycol als Zusatz zu einer Suspension von Arzneistoff-Partikeln organische Säuren, Salze, Anhydride, Aminosäuren und Peptide, Proteins einschließlich Fibrinogen, eine Vielzahl funktionell definierter Substanzen und Beschichtungen (US 2008/0118544). Nur wenige dieser Hilfsstoffe sind wirklich nützlich und oft nur für bestimmte Wirkstoffe und Beschichtungen. Viele der genannten Hilfsstoffe wirken zellmembranschädigend (Detergentien, amphiphile Substanzen), verhindern die rasche Aufnahme der Wirkstoffe in die Zellen oder sind selbst instabil. Für den Fachmann ist meist unvorhersehbar, welcher Hilfsstoff in welcher Dosis und mit welchem Wirkstoff nützlich ist.
In der WO2004/028610 wird einen Katheterballon der mit einem lipophilen Wirkstoff beschichtet ist beschrieben. Der Wirkstoff wird bei der Expansion des Katheterballons sofort freigesetzt. Hilfsstoffe, zum Beispiel Zucker oder Polyethylenglykol, können an der Beschichtungszusammensetzung hinzugefügt werden zur Verbesserung der Bindung zwischen dem Wirkstoff und dem Ballon.
Möglichkeiten zur Verzögerung der Freigabe der Wirkstoffe werden ausführlich beschrieben. Dagegen wurde der Methode der Beschichtung der Ballone bisher wenig Aufmerksamkeit gewidmet, obwohl diese außerordentlich wichtig ist, um den Anforderungen an ein reproduzierbar herstellbares Produkt gerecht zu werden und eine wirksame Dosis innerhalb von Sekunden bis maximal wenigen Minuten im Zielgewebe zu deponieren.
Für die Beschichtung der Ballone wurden bisher die folgenden Verfahren beschrieben:
WO 92/11890 A beschreibt die Verwendung von Mikrokapseln als Arzneistoffträger, die eine verzögerte Freisetzung der Wirkstoffe gewährleisten. Die Mikrokapseln werden durch ein Bindemittel, durch Verschmelzung mit der Ballonoberfläche oder in Vertiefungen der Ballonmembran festgehalten. Die Beladung erfolgt durch Sprühen oder Tauchen. Außer der Beschreibung der Vertiefungen in der Ballonmembran finden sich keine Hinweise darauf wie ein spezieller Wirkstoff in einer Weise auf den Ballon gebracht wird, dass dieser auf dem Weg durch die Einführschleuse und schnell fließendes Blut ausreichend fest haftet, um dann bei Ballonexpansion vollständig freigesetzt zu werden.
Nach WO 2004/006976 A werden Wirkstoffe durch Tauchen, Aufsaugen oder Sprühen auf eine raue oder strukturierte Ballonoberfläche aufgetragen, wobei sich der Ballon in expandiertem Zustand befindet. Eine hydrophile Schicht zwischen der Ballonmembran und dem lipophilen Arzneimittel soll die Ablösung des Wirkstoffs erleichtern.

In WO 00/21584 A wird beschrieben, dass wasserunlösliche Arzneistoffe durch Tauchen, Sprühen oder Tropfen mittels einer Pipette auf einen Ballon aufgetragen werden. Der Ballon ist mit einem Polymer beschichtet, das den Wirkstoff aufnimmt. Die Freigabe ist in der Beobachtungszeit von Minuten bis Stunden unvollständig.

Die an sich wünschenswerte Plazierung der Beschichtung unter den Längsfalten der Ballonkatheter wird in WO 2007090385 ausführlich und in mehreren Beispielen beschrieben. Die Wirkstoffzusammensetzungen werden mittels des Pipettier-, des Spritz- oder des Sprühverfahrens unter die Falten gebracht. Während eine präzise Beschichtung behauptet wird, belegen die Beispiele eine hohe Streuung der Dosierung.

US 2003/064965 A fordert eine rasche Freigabe von Arzneimittelzubereitungen von Ballonkathetern, wobei die Zubereitungen selbst eine kontrollierte (d.h. verzögerte) Freisetzung gewährleisten sollen. Zu diesem Zweck werden die Wirkstoffe in verkapselter Form z.B. als Liposomen, Kolloid, Mikropartikel, Aggregate oder Ausflockungen eingesetzt. Als Matrix werden Fibrin- oder Hydrogele oder auch Glucose vorgeschlagen. Eine poröse Schicht soll die Beschichtung schützen. Einen schützenden Schlauch über der Beschichtung beschreibt auch US 2006/002973 A. Die Zubereitungen werden durch Sprühen, Tauchen, Rollen, Bürsten, lösungsmittel-vermittelte Bindung oder Haftstoffe auf die Ballonmembranen aufgebracht.

Weiterhin wurden als Verfahren zur Beschichtung offenbart: Sprühen im Vakuum, auch mit Suspensionen oder Emulsionen (DE 10 2004 048 265 A), die Verwendung von Fetten und Ölen (US 2004/224003 A, WO 2003/039612 A), die Verwendung von Substanzen oder Bedingungen, die die Freisetzung des Arzneistoffs auslösen (WO 96/39949 A), die Verwendung von lipophilen Hydratations-Inhibitoren (WO 2005/089855 A), die Beschichtung von Ballonen mit vormontierten Stents (z.B. DE 10 2004 046 244 A; US 2005/0033417 A) und der Schutz der beschichteten Ballone durch Hüllen, die erst kurz vor dem Expandieren der Ballone zurückgezogen werden.

In EP 1 372 737 A und WO 2004/028582 A werden Verfahren offenbart, die unter anderem eine Beschichtung von Ballonkathetern mit bei Expansion der Ballone sofort bioverfügbaren lipophilen Wirkstoffen beschreiben. Die Beschichtung erfolgt durch Tauchen, Bestreichen, Sprühen oder mittels einer Volumenmeßeinrichtung.

Die Bedeutung der Gleichförmigkeit der Beschichtung der Oberfläche wird in WO 2004/006976 A durch Beschichtung der Ballone in expandiertem Zustand mit gleichartig von außen zugänglicher Oberfläche Rechnung getragen, in WO 2001/052772 A für Produkte unterschiedlicher Art durch Verwendung eines Vibrators während des Beschichtungsvorgangs.

Bisher hat sich mit Ausnahme der entsprechend EP 1 372 737 A und WO 2004/028582 A beschichteten Ballone keiner der beschriebenen Ballonkatheter im Hinblick auf eine Verbesserung des klinischen Erfolgs oder auch nur tierexperimentell im Hinblick auf die erwünschten biologischen und therapeutischen Zielgrößen als wirksam erwiesen. Die Beschichtungsmethoden sind nur vage beschrieben oder die beschriebenen Methoden führen zu Produkten mit erheblichen Mängeln.

Trotz sehr guter Wirksamkeit weisen die in den Patentschriften EP 1 372 737 A und WO 2004/028582 A beschriebenen Ballonkatheter Nachteile auf, die bei einem pharmazeutischen Produkt unerwünscht wären. Viele der in sämtlichen vorgenannten Patentschriften aufgeführten Wirkstoffe lassen sich mit Kathetern, die entsprechend den beschriebenen Methoden beschichtet sind, gar nicht oder mit ganz unbefriedigender Ausbeute an den Wirkort bringen oder es sind jedenfalls keine Verfahren offenbart, nach denen der Fachmann zu einem brauchbaren und dem Stand der Technik entsprechenden Produkt kommen kann. Die bisher beschriebenen Arzneimittel-Beschichtungen für Ballonkatheter sind entweder nicht ausreichend oder nicht ausreichend zuverlässig wirksam, unter anderem weil die Arzneistoffe zu ungleichmäßig verteilt sind, zu fest oder zu wenig fest an der Ballonmembran haften, sich zu rasch oder zu langsam auflösen oder sie enthalten Hilfsstoffe, die ihrerseits die Gefäßwand schädigen oder sind unnötig komplex aufgebaut, was zu Nachteilen im Hinblick auf die Herstellung, Reproduzierbarkeit, Haltbarkeit und Anwendung führt.

Ballonkatheter zur Übertragung von Wirkstoffen auf die Gefäßwand, ohne dass es gleichzeitig zu einer Überdehnung und Schädigung der Gefäßwand kommt sind bisher nicht beschrieben. Werden die gebräuchlichen Angioplastie-Ballone in einem Durchmesser gewählt, der nicht zu einer Dehnung des Gefäßes führt, liegt deren Membran nur stellenweise der unregelmäßig geformten Gefäßwand an und überträgt nur dort den Arzneistoff.

### Definitionen

- Medizinprodukt:: Instrumente zur Behandlung oder Vorbeugung von Erkrankungen, ggf. unterstützt durch pharmakologisch wirksame Substanzen;
- Ballonkatheter:: Katheter mit einem expandierbaren distalen Segment;
- Ballonmembran:: Membran oder Ballonmembran bezeichnet die Außenhülle des Katheterballons, welche mit der Gefäßwand in Berührung kommt; bevorzugt sind glatte Membranen und Membranen, die in gefaltetem Zustand beschichtet werden; die gebräuchlichen Ballonkatheter weisen glatte Ballonmembranen auf. Die Strukturierung oder Aufrauung von Ballonmembranen erfordert besondere Maßnahmen bei der Herstellung.
- Stent:: Röhrenförmige Struktur zur Deposition in Hohlräumen oder Geweben (Gefäßstütze);
- Wirkstoff:: Biologisch oder medizinisch wirksame Substanz; bevorzugt sind Arzneistoffe, d.h. in zugelassenen Arzneimitteln enthaltene Wirkstoffe;
- Hilfsstoff:: Substanz ohne beabsichtigte biologische Wirkung;
- Matrixsubstanz:: Substanz, die einen Wirkstoff umschließt oder anderweitig festhält; die Matrix kann selbst eine biologische Wirkung entfalten;
- Lipophile Substanz:Affinität: zu Fetten; gemessen als Verteilungskoeffizient zwischen einem fettlösenden und einem wässrigen Lösungsmittel;
- Hydrophile Substanz:: Affinität zu Wasser; gemessen als Verteilungskoeffizient zwischen einem fettlösenden und einem wässrigen Lösungsmittel;
- Wasserlösliche und/oder hydrophile Wirkstoffe:: Biologisch wirksame Substanzen, die sich als solche oder in Form eines beliebigen Salzes zu mindestens 1 mg/ml (bevorzugt zu 5 mg/ml, besonders bevorzugt zu 20 mg/ml) in Wasser oder einem wässrigen Medium wie Plasma oder Blut lösen oder einen Verteilungskoeffizienten Butanol/Wasser von kleiner als 0,5 aufweisen
- Schlecht wasserlöslich:: Unter dem Begriff "schlecht wasserlöslich" soll eine Löslichkeit des betreffenden Stoffes in Wasser von weniger als 5 mg/ml bevorzugt weniger als 1 mg/ml verstanden werden.
- Hydrophile Lösungsmittel:: Lösungsmittel in denen sich bei Raumtemperatur wenigsten 1 vol% Wasser löst, bevorzugt 10 vol %.
- Sofortige Bioverfügbarkeit:: Übertragung des Wirkstoffs in das Gewebe während der kurzen Zeit der Ballondilatation, ohne dass die Auflösung der Wirksubstanz oder deren Freisetzung im Gewebe durch besondere Maßnahmen wie Verkapselung verzögert wird;.
- Sofort freigesetzt:: Bedeutet, dass eine wirksame Dosis des Wirkstoffs bei der Expansion des Ballons innerhalb max. einer Minute an die Umgebung abgegeben wird. Der Wirkstoff kann z.B. in partikulärer Form abgegeben und im Laufe längerer Zeit durch Auflösung wirksam werden;
- Niedermolekular:: Substanzen mit einem Molekulargewicht von kleiner 5000 D, bevorzugt < 2000 D, besonders bevorzugt < 1000 D;
- Hydrophile / hydrophilisierte Ballonmembran:: hydrophile Membranen bestehen aus mit Wasser oder hydrophilen Lösungsmitteln benetzbarem Material, hydrophilisierte Membranen sind Ballonmembranen beispielsweise aus Nylon, deren Oberfläche durch eine nachträgliche Behandlung in einen mit Wasser oder anderen hydrophilen Lösungsmitteln benetzbaren Zustand verändert wurde. Hydrophile bzw. hydrophilisierte Membranen sind nicht mit Membranen zu verwechseln, die mit einer zusätzlichen hydrophilen Schicht versehen wurden.
- Hydrophil beschichtete Ballonmembran:: Membran, die eine zusätzlich aufgebrachte Schicht enthält, die ihrerseits mit Wasser benetzbar ist.
- Offen an der: Oberfläche: Arznei- oder Hilfsstoffe, die nicht in die Ballonmembran oder in fest mit der Ballonmembran verbundene Beschichtungen inkorporiert sind, beispielsweise nicht in polymere, wasserunlösliche Beschichtungen wie insbesondere sich nicht ablösende Hydrogele. Offen an der Oberfläche schließt Beschichtungen ein, die durch die Falten der Ballonmembran in gefaltetem Zustand abgedeckt werden.
- Feste Haftung:: Gefaltete Ballonkatheter (Orbus IX, Bavaria Medizin Technologie, Oberpfaffenhofen, Deutschland, SeQent, BBraun, Melsungen, Deutschland Ballongröße 3.5 mm Durchmesser, 20 mm Länge oder vergleichbare Produkte anderer Hersteller) werden in gefaltetem Zustand mit 3 µg Wirkstoff / mm² nach dem unten beschriebenen Dosierverfahren beschichtet. Die Ballone werden trocken expandiert und in einem Glas 5 sec geschüttelt: Es bleiben mehr als 75 % der Dosis an dem Ballon haften.
- Leicht flüchtig:: Lösungsmittel mit einem Siedepunkt unter 300°C, bevorzugt unter 160°C, besonders bevorzugt unter 100°C.

### Beschreibung der Erfindung

Die Erfindung hat zur Aufgabe, verbesserte Medizinprodukte wie z.B. Ballonkatheter zur Verfügung zu stellen, die eine zuverlässigere lokale Behandlung von erkrankten Geweben ermöglichen, neue Anwendungen erschließen und den Einsatz hydrophiler, gut wasserlöslicher Wirkstoffe als Beschichtungsbestandteil ermöglichen.
Insbesondere ist Aufgabe der vorliegenden Erfindung, einen Ballonkatheter bereitzustellen, der nicht zu einer Dehnung oder Überdehnung des Gefäßes führt und dennoch zur Behandlung oder Prophylaxe von Erkrankungen der Gefäßwand eine ausreichende Menge an Wirkstoff freisetzt.

Diese Aufgabe wird durch die unabhängigen Patentansprüche der vorliegenden Erfindung gelöst. Weitere vorteilhafte Ausgestaltungen sind der Beschreibung, den Beispielen und den abhängigen Patentansprüchen zu entnehmen. Dazu werden neuartige Ballonkatheter spezieller Bauart und Methoden der Beschichtung von Ballonkathetern in ausreichend detaillierter Form offenbart. Ferner können folgende Wirkstoffe und Hilfsstoffe erfindungsgemäß eingesetzt werden.

### Wirkstoffe und Hilfsstoffe

Als Wirkstoffe werden antiproliferative, antiinflammatorische, antiphlogistische, antihyperplastische, antineoplastische, antimitotische, zytostatische, zytotoxische, antiangiogene, antirestenotische, mikrotubuli-inhibierende, antimigrative oder antithrombotische Wirkstoffe bevorzugt.

Beispiele für antiproliferative, antiinflammatorische, antiphlogistische, antihyperplastische, antineoplastische, antimitotische, zytostatische, zytotoxische, antiangiogene, antirestenotische, mikrotubuli-inhibierende, antimigrative oder antithrombotische Wirkstoffe sind:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Arsentrioxid und andere Arsenverbindungen, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1 a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Wismuth und Wismuthverbindungen oder -chelate, Irinotecan, Topotecan, Hydroxycarbamid, Miltefosin, Pentostatin, Pegasparase, Exemestan, Letrozol, Formestan, SMC-Proliferation-Inhibitor-2ω, Mitoxanthrone, Mycophenolatmofetil, c-myc-Antisense, b-myc-Antisense, ß-Lapachon,Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, die Muskelzellproliferation hemmende monoklonale Antikörper, bFGF-Antagonisten, Probucol, Prostaglandine, 1-Hydroxy-11-Methoxycanthin-6-on, Scopolectin, NO-Donatoren, Pentaerythrityltetranitrat, Syndnoeimine, S-Nitrosoderivate, Tamoxifen, Staurosporin, ß-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel, Derivate von Paclitaxel, 6-α-Hydroxy-Paclitaxel, 2'-Succinylpaclitaxel, 2'-Succinylpaclitaxeltriethanolamin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere, Kohlensuboxids (MCS), macrocyclische Oligomere von Kohensuboxid, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, in Virenüberträger inkorporierte Nukleinsäuren, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Enoxoparin, desulfatiertes und N-reacetyliertes Heparin, Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon a, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Rapamycin in Kombination mit Arsen oder Arsenverbindungen oder Komplexen, Somatostatin, Tacrolimus, Roxithromycin, Troleandomycin, Simvastatin, Rosuvastatin, Vinblastin, Vincristin, Vindesin, Thalidomid, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin, Fasudil.

Bevorzugte Wirkstoffe, welche auf einem Katheterballon aufgetragen sein können, sind Paclitaxel und andere Taxane, Rapamycin und andere mTOR (mammalian target of rapamycin)-Hemmer Methotrexinsäure Arsen oder Arsenverbindungen, Wismuth oder Wismuthverbindungen, oder Thalidomid.

In einer weiteren bevorzugten Ausführungsform liegt der mindestens eine Wirkstoff als schlecht wasserlöslicher Neutralstoff, als schlecht wasserlösliches Salz oder als schlecht wasserlösliche Säure oder schlecht wasserlösliche Base vor.

Als hydrophiler Hilfsstoff werden vorzugsweise flüchtige hydrophile Lösungsmittel oder hydrophile Lösungsmittelgemische sowie nicht flüchtige Substanzen ohne eine bei der Art der Verabreichung beabsichtigte biologische Wirkung wie Zucker, Zuckeralkohole, Aminosäuren, Fette, anorganische oder organische Salze und/ oder für die intravasale Applikation geeignete Kontrastmittel oder Farbstoffe eingesetzt. Erfindungsgemäß wird Harnstoff als Hilfsstoff zugegeben. Weitere Hilfstoffe können Ascorbinsäure, Polyethylenglykol 8000 und trotz geringer Wasserlöslichkeit auch Triglyceride, insbesondere bei Raumtemperatur feste Triglyceride wie Trimyristin sein.

### Ballonkatheter zur Beschichtung:

In den oben genannten Patentschriften sind gebräuchliche glattwandige Ballonkatheter für die perkutane transluminale Angioplastie beschrieben, bestehend aus unterschiedlichen Materialien wie z.B. Nylon, PEBAX, Polyethylen und viele andere, die in der DE 10 2004 046 244 und anderen Patentschriften offenbart sind, Ballonkatheter mit Furchen oder Poren, in denen Wirkstoffe platziert werden, oder Ballonkatheter mit strukturierten und aufgerauten Membranen. Ziel der Strukturveränderungen im Sinne einer Oberflächenvergrößerung ist es die Beladbarkeit mit Wirkstoffen zu vergrößern oder die Haftung der Wirkstoffe an den Ballonen zu verbessern. In WO 2004/006976 werden darüber hinaus Ballone mit einer zusätzlichen hydrophilen Schicht beschrieben. Die Ballone dieser Katheter sind bis zu einer vorgegebenen Größe expandierbar und möglichst druckresistent, um stenotische Arterien wieder auf ihren ursprünglichen Durchmesser aufzuweiten.

Die Strukturierung der Oberflächen hat allerdings den Nachteil, die Wirkstoffabgabe bei Expansion der Ballone in den Gefäßen zu verzögern. Die Ballone blockieren im expandierten Zustand den Blutfluß durch das behandelte Gefäß vollständig. Eine Blockade des Blutflusses wird insbesondere in den Koronararterien nur für sehr kurze Zeit toleriert. In dieser Zeit muß die wirksame Dosis freigesetzt werden. Jede Verzögerung der Ablösung des mindestens einen Wirkstoffs von der Ballonmembran ist von Nachteil.

Es wurde überraschend gefunden, dass sich hydrophile oder hydrophilisierte Ballonmembranen mit Wirkstoffen reproduzierbarer und gleichmäßiger beschichten lassen, zudem ein breiteres Spektrum an Lösungsmitteln für die Beschichtung erlauben und der mindestens eine Wirkstoff ausgezeichnet an der Ballonmembran haftet.. Dies gilt insbesondere für den Fall, dass Ballone im bereits gefalteten Zustand beschichtet werden sollen. Hydrophile Ballonmembranen sind bekannt und werden verwendet, um die Gleitfähigkeit der Katheter vor der Expansion der Ballone zu verbessern.

Somit betrifft die vorliegende Erfindung einen Ballonkatheter umfassend einen Katheterballon mit einer Ballonmembran, wobei die Ballonmembran hydrophil oder hydrophilisiert ist und/oder die Oberfläche der Ballonmembran eine hydrophile Beschichtung trägt. Diese hydrophile Beschichtung haftet vorzugsweise fest an der Ballonoberfläche, d.h. ist fest mit der Ballonoberfläche verbunden und löst sich bei der Dilatation des Katheterballons nicht ab.

Die vorliegende Erfindung betrifft weiterhin Ballonkatheter umfassend einen Katheterballon mit einer hydrophilen oder hydrophilisierten Ballonmembran, wobei die Ballonmembran mit mindestens einem offen an deren Oberfläche aufliegenden Wirkstoff und Harnstoff in einer Weise beschichtet ist, dass der mindestens eine Wirkstoff bei der Expansion des Katheterballons sofort freigesetzt wird. Ferner kann der Katheterballon zusätzlich mit beliebigen Hilfsstoffen beschichtet sein.

Bevorzugte erfindungsgemäße Katheterballons weisen daher zwei Beschichtungen auf, eine untere fest anhaftende hydrophile Beschichtung und eine äußere ablösbare Beschichtung aus einem Wirkstoff und Harnstoff oder einer Zusammensetzung enthaltend mindestens einen Wirkstoff und Harnstoff.

Des weiteren ist bevorzugt, wenn die in der Regel lipophile Ballonoberfläche mittels aktiviertem Sauerstoff behandelt wird, um diese hydrophil zu machen. Die hydrophile Ballonmembran oder konkreter gesagt die hydrophile Oberfläche der Ballonmembran, d.h. die hydrophile Oberfläche des Ballons kann durch eine hydrophile Beschichtung an sich lipophiler Ballonmembranen bzw. Ballonoberflächen oder - zum Zwecke der Beschichtung bevorzugt - durch chemische Veränderung (z. B. durch Reaktion mit aktiviertem Sauerstoff) einer lipophilen Membran erzeugt werden.

Die hydrophilen Katheterballons lassen sich selbst mit einfachen Verfahren wie dem Tauchen gut reproduzierbar mit einer Beschichtungszusammensetzung beschichten, so dass der Wirkstoffgehalt auf dem mit mindestens einem Wirkstoff beschichteten Katheterballon eine Standardabweichung vom Mittelwert von weniger als 20%, vorzugsweise weniger als 15%, weiter bevorzugt weiniger als 10% und insbesondere bevorzugt weniger als 5% aufweist.

Durch die Beschichtung mit Wirkstoffen oder Hilfsstoffen kann die verbesserte Gleitfähigkeit der hydrophilen Ballone verloren gehen, jedenfalls wenn sich die Beschichtung auch außen auf dem nicht expandierten Ballon befindet. Hydrophile Ballone haben den Nachteil, dass sie bei Expansion in verengten Arterien leichter aus der gewünschten Position rutschen. Dieser Nachteil wird durch die Beschichtung mit Arznei- und Matrixstoffen nach unserer Beobachtung weitgehend aufgehoben, da die zunächst nicht im umgebenden Medium gelöste Beschichtung die Reibung zwischen Ballon und Arterienwand deutlich erhöht.

Die gebräuchlichen Angioplastie-Ballone sollen die Gefäße nicht überdehnen. Sie erreichen daher bei geringem Druck einen bestimmten Durchmesser, der durch Druckerhöhung nicht wesentlich zu steigern ist.

Eine weitere nützliche Modifikation der Ballonmembran betrifft deren mechanische Eigenschaften: Zur Übertragung von Wirkstoffen auf die Gefäßwand ohne deren Überdehnung werden Membranen gewählt, die weich (,compliant') und bei geringem Druck dehnbar sind oder den Gefäßdurchmesser wesentlich überschreiten. Wesentlich überschreiten bedeutet, dass der Ballondurchmesser den Referenzdurchmesser des Gefäßes bevorzugt um mindestens 20 %, besonders bevorzugt mehr als 30% überschreitet, wobei der Ballon möglichst mit nicht mehr als ca. 2 000 hPa aufgedehnt werden soll. Diese Ballone sind nicht dafür vorgesehen, dass Lumen des Gefäßes durch den Druck auf die Gefäßwand wesentlich zu erweitern. Eine wesentliche Erweiterung des Lumens ist insbesondere die Beseitigung eines Verschlusses oder einer hochgradigen Stenose oder die Erweiterung des Lumens um mehr als 30% des Referenzdurchmessers des Gefäßes. Die Membraneigenschaften können durch dem Fachmann bekannte Wahl der Zusammensetzung der Membran und/oder deren Wandstärke und Faltung erzielt werden. Die Ballone können einen vergleichsweise geringen Berstdruck haben, z.B. gleich oder kleiner 10.000 hPa (9,87 atm; [1 atm = 1013 hPa]), bevorzugt gleich oder kleiner 5.000 hPa (4,93 atm), da sie nicht mit hohem Druck expandiert werden. Bevorzugte Expansionsdrucke liegen vorzugsweise unter 4.000 hPa (3,95 atm), weiter bevorzugt unter 2.000 hPa (1,97 atm) und noch weiter bevorzugt unter 1.000 hPa (0,97 atm) über Normaldruck. Besonders bevorzugt sind Drucke zwischen 2.000 hPa (1,97 atm) und 200 hPa (0,20) über normal. Bevorzugt sind Katheter zur Behandlung von Arterien, Venen oder Dialyseshunts mit Ballonabmessungen von einem Durchmesser zu Längenverhältnis von kleiner 0.2, besonders bevorzugt mit einem Durchmesser zu Längenverhältnis von kleiner 0.1.
Die beschriebenen Ballone sind nicht mit Ballonen zu verwechseln, die z.B. aus Silikon oder Latex bestehen, meist rundlich sind und zur Fixierung von Kathetern in Hohlräumen wie der Harnblase benutzt werden, ohne den betreffenden Hohlraum vollständig auszufüllen.

Erfindungsgemäß sind ferner Ballonkatheter bevorzugt, welche bereits bei geringem Druck ihren Maximaldurchmessen im expandierten Zustand erreichen und dennoch eine gewisse Flexibilität besitzen, um sich einen unebenen Gefäßwand anzupassen. Somit ist bevorzugt, wenn sich der Radius des Katheterballons nach vollständiger Entfaltung durch Druckerhöhung um mehr als 15%, vorzugsweise mehr als 30% und insbesondere bevorzugt mehr als 60% vergrößert. Die Druckerhöhung findet durch Einleiten von Gas (z.B. Kohlendioxid) oder einer Flüssigkeit wie beispielsweise einem Kontrastmittel im Inneren des Katheterballons in üblicher Weise statt.

Bevorzugt sind des weiteren Ballonkatheter bei denen sich der Radius des Katheterballons nach vollständiger Entfaltung durch Druckerhöhung im Inneren des Katheterballons um mehr als 15%, vorzugsweise mehr als 30% und insbesondere bevorzugt mehr als 60% vergrößert.

Eine weitere Ausführungsform der vorliegenden Erfindung ist auf einen Ballonkatheter mit mindestens einem offen an der Oberfläche aufliegenden Wirkstoff der bei der Expansion des Katheterballons sofort freigesetzt wird gerichtet, wobei sich der Radius des Katheterballons nach vollständiger Entfaltung durch Druckerhöhung im Inneren des Katheterballons um mehr als 15%, vorzugsweise mehr als 30% und insbesondere bevorzugt mehr als 60% vergrößert.

Der Wirkstoff oder die Wirkstoffe und ggf. weitere Hilfsstoffe haften an der Ballonmembran und/oder sind durch deren Struktur oder die Faltung der gebrauchsfertigen Ballone trotz der geringen Festigkeit der Membran überraschend gut vor dem vorzeitigen Ablösen geschützt. Die Struktur der Ballonmembran im kontrahierten oder Ruhezustand, d.h. ohne dass der Ballon expandiert wird, kann beliebig geformte Nischen, Vertiefungen und Erhebungen oder Falten beinhalten, die sich wegen der Biegsamkeit und Dehnbarkeit der Membran bei der Expansion mit geringem Druck glätten. Diese Ballone sind besonders vorteilhaft zur Behandlung von Gefäßveränderungen, die den Blutfluß nicht wesentlich einschränken, d.h. die das freie Gefäßlumen um weniger als 50% verengen. Sie erlauben die Behandlung von wenig druckresistenten Gefäßen, da sie sich bei geringem Druck auch einer unregelmäßig verlaufenden Gefäßwand anlegen. Insbesondere sind die erfindungsgemäßen Ballonkatheter zur lokalen Behandlung und Prophylaxe von Gefäßerkrankungen geeignet und insbesondere von entzündlichen Gefäßveränderungen, vulnerablen Plaques, mechanisch oder chirurgisch vorbehandelten Gefäßabschnitten, langstreckigen Läsionen ohne die Notwendigkeit (erneuter) Aufdehnung auch kleiner Gefäße, welche für einen Stent nicht zugänglich sind.
Die erfindungsgemäßen Ballonkatheter eignen sich hervorragend zur Behandlung von Gefäßwandveränderungen, die den Blutfluß nicht wesentlich einschränken.

### Beschichtung

Eines der bisher ungelösten Probleme ist es, eine hinreichend genaue Dosis eines Wirkstoffs ausreichend gleichmäßig auf einer Ballonoberfläche zu verteilen. An die Verabreichung von Arzneistoffen werden hohe Anforderungen bezüglich der Dosiergenauigkeit in der Arzneiform, das ist in diesem Falle die Ballonbeschichtung, gestellt. Während exakte Dosierverfahren aus der Pharmazie bekannt sind, besteht bei den meisten pharmazeutischen Anwendungen keine Notwendigkeit, Wirkstoffe gleichmäßig auf einer Oberfläche zu verteilen. Zudem arbeiten die in der Pharmazie und Biochemie üblichen Dosiergeräte meist mit wässrigen Lösungen, bei denen der Dampfdruck die Dosierung bei Raumtemperatur nicht wesentlich erschwert.

In den unten beschriebenen Patentschriften finden sich einige vage Hinweise wie das Problem zu lösen sein könnte, jedoch wurde die Bedeutung des Problems nicht erkannt, insbesondere wurden aber keine Verfahren beschrieben, die es dem Fachmann ermöglichen würden, Ballone auf wirtschaftliche und reproduzierbare Weise so zu beschichten, dass die Produkte den Arzneistoff am Wirkort rasch und vollständig freisetzen und zuverlässig wirksam sind.

Das im EP 1 372 737 A und WO 2004/028582 A offenbarte Beschichtungsverfahren durch wiederholtes Tauchen gebräuchlicher, gebrauchsfertig gefalteter Ballone in wenig visköse Lösungen lipophiler Arzneistoffe und geeigneter Hilfsstoffe hat zu einer für die medizinische Anwendung zunächst ausreichend reproduzierbaren Dosis auf den Ballonen geführt. Eine wichtige Erkenntnis war, dass es trotz inhomogener Wirkstoffverteilung in radialer Richtung der Ballone bedingt durch die Faltung bei Expansion der Ballone zu einer gleichmäßigen Verteilung des mindestens einen Wirkstoffs auf der Gefäßwand kommt (Scheller B, Speck U, Böhm M. Prevention of restenosis - is angioplasty the answer? Heart 2007;93:539-541). Eine Reihe von Nachteilen war bei der routinemäßigen Anwendung zur Produktion dennoch offensichtlich. Das Verfahren ist unbequem und aufwändig anzuwenden, da es wiederholtes Tauchen mit dazwischen liegenden Trocknungsvorgängen erfordert. Die an den Ballonen haftende Menge Wirkstoff wird von einer Vielzahl, nicht immer kontrollierbarer Faktoren bestimmt. Während weitgehend gleichartige Ballone einer Charge meist befriedigend reproduzierbar beschichtet werden konnten, traf dies für Chargen aus verschiedenen Herstellungsgängen nicht immer zu. Ein weiteres schwierig zu lösendes Problem bei einem Tauchverfahren ist die longitudinale Wirkstoffverteilung. Insbesondere besteht die Möglichkeit, dass der proximale Ballonabschnitt nicht ausreichend beladen wird. Schließlich sind bei dem genannten Verfahren Maßnahmen erforderlich, um das Eindringen der wenig viskosen Lösung in das zentrale Lumen des Katheters zu verhindern.

Die übrigen vorbekannten Beschichtungsprozesse liefern noch ungünstigere Ergebnisse: Eine Beschichtung expandierter Ballone hat zur Folge, dass die Ballone mit Beschichtung gefaltet werden müssen. Dies ist im Hinblick auf die aufgetragene Dosis nur dann einigermaßen verlustfrei realisierbar wenn die Beschichtung fest haftet. Eine fest haftende Beschichtung wird aber während der kurzen Zeit des Kontaktes zwischen Ballonmembran und Gefäßwand nicht ausreichend freigesetzt. Beim Beschichten gefalteter Ballone durch Sprühen befindet sich der Wirkstoff nur oberflächlich auf dem Ballon, was zu erhöhten Verlusten beim Einführen der Ballonkatheter durch Einführungsschleusen, Führungskatheter und vorgeschaltete Blutgefäße führt. Sprühen, Bestreichen und Pipettieren gewährleisten weder eine reproduzierbare, genau vorherbestimmbare Dosis noch eine gleichmäßige Verteilung des mindestens einen Wirkstoffs auf den Kathetern. Mit den gebräuchlichen Pipetten ist die exakte Abmessung der benötigten sehr kleinen Volumina von bevorzugten leicht flüchtigen Lösungsmitteln ebenso schwierig wie die gleichmäßige Verteilung der Lösung auf dem Ballon.. Der Vorteil der in der WO 2007/090385 beschriebenen Verfahren ist die Platzierung der Wirkstoffe unter den Falten der Ballone.

Ein erfindungsgemäßes Verfahren zur Beschichtung von Medizinprodukten oder Teilen davon, wie z.B. Ballonen am distalen Ende von Kathetern umfasst die folgenden Schritte:
a) Bereitstellung eines Katheterballons,
b) Bereitstellung einer Mikrodosiereinheit enthaltend eine Beschichtungszusammensetzung die nicht im Kontakt mit einer Gasphase steht,
c) verlustfreie und gleichmäßige Beschichtung des Katheterballons mit der Beschichtungszusammensetzung unter Verwendung der Mikrodosiereinheit.

Bei der Beschichtungszusammensetzung handelt es sich in der Regel um eine Beschichtungslösung oder eine Beschichtungsflüssigkeit, wobei aber auch ein Gel oder eine Suspension, Emulsion, Dispersion oder Aufschlämmung verwendet werden kann.

Wichtig ist bei der Beschichtung, dass das Lösungsmittel der Beschichtungslösung nicht verdunsten kann bevor es auf den Ballon aufgetragen wurde. Daher sollte das Lösungsmittel nicht im Kontakt mit einer Gasphase stehen, deren Volumen einen Einfluß auf die abgegebene Dosis nehmen kann.

Der Katheterballon wird vorzugsweise während der Beschichtung horizontal gelagert und um seine Längsachse gedreht, während sich die Mikrodosiereinheit entlang der Längsachse des Katheterballons hin und her bewegt, um so eine vollständige Beschichtung des gefalteten oder nicht vollständig entfalteten Katheterballons zu gewährleisten.

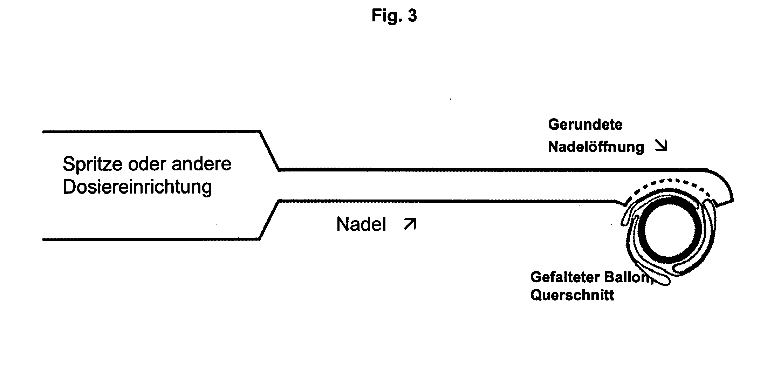

Als Mikrodosiereinheit kann eine Spritze (s. Fig. 3), Kanüle, ein Schlauch oder eine andere Vorrichtung verwendet werden, welche präzise genug für die Abgabe der benötigten kleinen Mengen auf den Katheterballon ist und den Katheterballon bei der Beschichtung nicht beschädigt und vorzugsweise nicht einmal berührt.

Als Lösungsmittel für die Beschichtungszusammensetzung werden vorzugsweise leicht flüchtige Lösungsmittel oder Chlorverbindungen oder Fluorverbindungen mit einem Siedepunkt unter 300°C, bevorzugt unter 100°C, eingesetzt. Des weiteren können hydrophile Lösungsmittel oder Gemische mindestens eines Lösungsmittels oder hydrophilen Lösungsmittels mit Wasser verwendet werden.

Die Ballone werden bevorzugt in gefalteter Form beschichtet, können aber auch mit entsprechend angepasstem Gerät in beliebiger anderer Form beschichtet werden.

Um eine gleichmäßige Beschichtung zu erreichen, sollte die gesamte Ballonmembran von proximal bis distal und in allen Falten während der Beschichtung gleichzeitig mit der Beschichtungszusammensetzung benetzt sein, jedoch ohne dass diese abtropft.

Als Beschichtungszusammensetzung kann auch ein Gel verwendet werden. Dabei kann der enthaltene mindestens eine Wirkstoff selbst als Gelbildner fungieren oder an der Gelbildung teilnehmen. Der Wirkstoff selbst fungiert als Gelbildner, wenn eine gelartige Beschichtungszusammensetzung erhalten wird, ohne dass neben dem Wirkstoff weitere gelbildende Substanzen anwesend sind.

Zudem ist bevorzugt, wenn der mindestens eine Wirkstoff in schlecht wasserlöslicher Form auf den Katheterballon aufgetragen wird.

Als Alternative kann der mindestens eine Wirkstoff, der eventuell gut wasserlöslich, d.h. hydrophil ist, nach dem Auftragen auf den Katheterballon auch in eine schlecht wasserlösliche Form überführt werden. Dies kann z.B. durch Komplexierung mit Cyclodextrinen oder Salzbildung geschehen. Die Herstellung eines schlecht wasserlöslichen Salzes als auch die Auswahl eines Gegenions oder Komplexbildners gehören zum Standardwissen eines Fachmanns und können durch einfache Löslichkeitsversuche herausgefunden werden.

Eine bevorzugte Ausführungsform des erfindungsgemäßen Beschichtungsverfahrens wird im folgenden beschrieben und umfasst:
A) Die Bereitstellung:
   1) definierter Ballonkatheter oder geeigneter den Ballon enthaltender Bauteile, wobei sich der Ballon bevorzugt im gefalteten Zustand oder in einem Zustand mit vorgeformten, aber nicht endgültig festgepreßten Falten befindet
   2) einer Einrichtung zur Halterung des Ballons bevorzugt in horizontaler Lage, wobei der Ballon in einer bevorzugten Ausführung um seine Längsachse rotiert werden kann
   3) einer Mikro-Volumenmeßeinrichtung zur Abgabe von Lösungen, enthaltend bevorzugt leicht flüchtige organische Lösungsmittel, bei der das abzugebende Volumen sich nicht mit einer Gasphase in Kontakt befindet, deren Volumen einen Einfluß auf die abgegebene Dosis nehmen kann.
   4) eines Übertragungselements zur Überleitung der Flüssigkeit von der Volumenmeßeinrichtung auf den Ballon
   5) einer Lösung mit mindestens einem Wirkstoff und optional einem oder mehreren Hilfsstoffen.
B) Die Arbeitsschritte
   1) Berechnung des für die Beschichtung in der erwünschten Dosis notwendigen Volumens der Lösung mit Hilfe der bekannten Ballonoberfläche in mm² und der Konzentration des Wirkstoffs in der Lösung
   2) Einsetzen des Katheters oder des den Ballon enthaltenden Bauteils des Katheters in die Halterung
   3) Kalibrierung der Volumenmeßeinrichtung auf das errechnete Volumen mit dem verwendeten Lösungsmittel
   4) Gasblasenfreie Füllung der Volumenmeßeinrichtung mit der Beschichtungslösung
   5) Langsame kontinuierliche Drehung des Ballons um seine Längsachse
   6) Positionierung des Übertragungselements mit der Öffnung, durch die die Lösung austritt, auf dem Ballon oder knapp über dem Ballon, oder direkt unter dem Ballon oder seitlich
   7) Übertragung des vorgesehenen Volumens der Beschichtungslösung auf den Ballon während das Übertragungselement mit gleichmäßiger Geschwindigkeit auf dem zylindrischen Teil des Ballons in Richtung der Längsachse hin- und her bewegt wird. Die Geschwindigkeit der Übertragung der Lösung ist bevorzugt so einzustellen, dass alle Teile des Ballons gleichzeitig mit der Flüssigkeit benetzt sind, ohne dass sich Tropfen an dem Ballon bilden, die herunterfallen.

Während die Volumenmeßeinrichtung eine genaue Dosis auf dem Ballon gewährleistet unabhängig vom Ballonmaterial, dessen Oberflächenstruktur (glatt oder strukturiert, fertig oder lose gefaltet oder teils oder ganz expandiert, der Größe und Beschaffenheit der Ballone sowie der individuellen Ballonchargen, bewirkt die Bewegung von Ballon und Überleitungselement verbunden mit der vollständigen Durchnässung des Ballons mit der Beschichtungslösung eine überraschend gleichmäßige Verteilung selbst auf lang gestreckten Ballonen.

Nach dem Beschichten können die Ballone unter geeigneten Bedingungen fertig gefaltet und/oder getrocknet werden, es können Stents montiert werden und die Katheter werden in gebräuchlicher Weise verpackt und sterilisiert.

Das oben beschriebene Prinzip der Beschichtung lässt sich vom Fachmann mit Gegenständen und Geräten unterschiedlicher Art auf unterschiedliche Weise realisieren und den zu beschichtenden Gegenständen anpassen. Es zeichnet sich durch Genauigkeit der Dosierung und Platzierung, Gleichmäßigkeit der Verteilung der Beschichtung auf der Oberfläche des zu beschichtenden Bereiches einschließlich des Eindringens in Falten und andere nicht frei zugängliche Strukturen aus. Es ist einfach und wirtschaftlich in der Handhabung, da Material- und Zeitbedarf minimal sind und der Prozeß leicht kontrollierbar und automatisierbar ist. Insbesondere wird der Verlust von Beschichtungszubereitung in Behältnissen und durch unerwünschte Verteilung derselben auf dem Medizinprodukt oder in dessen Umgebung vermieden. Eine Veränderung der Beschichtungszubereitung bereits vor dem Auftragen auf das Medizinprodukt durch vorzeitiges Verdampfen flüchtiger Lösungsmittel ist ausgeschlossen.

### Beschleunigte Ab- und Auflösung lipophiler Wirkstoffe

In der EP 1 372 737 A und WO 2004/028582 A, in dem US-Patent Nr. 6,306,166 und anderen Patenschriften werden eine Reihe von Lösungsmitteln für die Beschichtung von Medizinprodukten mit Arzneimitteln beschrieben. Es wurde überraschend gefunden, dass sich lipophile Arzneimittel, beispielsweise Paclitaxel und andere Taxane, aber auch Rapamycin und verwandte Stoffe in wässrigem Medium, Blut oder Gewebe besonders gut von der Ballonoberfläche ablösen und in Lösung gehen, wenn die Beschichtung der Medizinprodukte mit Lösungen der Substanzen in Chloroform oder Dichlormethan oder anderen leicht flüchtigen Chlor- oder Fluorverbindungen oder deren Gemischen erfolgt.

Weitere geeignete Maßnahmen zur Beschleunigung der Ablösung lipophiler und/oder schwer wasserlöslicher Wirkstoffe sind die Verwendung von hydrophilen flüchtigen organischen Lösungsmitteln, insbesondere Methanol, Ethanol, Propanol, Ameisensäure, Essigsäure, Tetrahydrofuran (THF), Aceton, Butanon, 3-Pentanon, Carbonsäureester insbesondere Ameisensäuremethylester, Ameisensäureethylester, Essigsäuremethylester, Essigsäureethylester etc. und deren Gemische mit Wasser. Eine besonders bevorzugte Form der Beschichtung mit beispielsweise Paclitaxel verzichtet auf jede nachträgliche Beschichtung der ursprünglichen Ballonmembran mit anderen Polymeren, Hydrogelen oder sonstigen Trägerschichten für die Arzneistoffe, alle Zusatzstoffe und komplexen Lösungsmittelgemische. Solche Beschichtungen haben sich bisher als weitgehend unwirksam erwiesen (Scheller B, Speck U, Abramjuk C, Bernhardt U, Böhm M, Nickenig G: Paclitaxel balloon coating - a novel method for prevention and therapy of restenosis. Circulation 2004; 110: 810-814, WO 2004/028582, Beispiel 7; Cremers B, Biedermann M, Mahnkopf D, Böhm M, Scheller B.Paclitaxel-beschichtete PTCA-Katheter: Gibt es Unterschiede? Einfluss von PACCOCATH®- und DIOR®-Ballonkathetern auf die Neointimaproliferation an Schweinekoronarien. Clin Res Cardiol 2008; 97- Suppl 1: V1742).

Überraschenderweise lässt sich die Kristallstruktur und die Haftung von Paclitaxel an der Ballonmembran durch den Zusatz geringer Anteile Wasser zu einer Lösung von Paclitaxel in beispielsweise Isopropanol, Tetrahydrofuran, Dimethylformamid oder Essigsäure oder Mischungen enthaltend eines dieser Lösungsmittel sehr genau steuern. Bevorzugt sind Lösungsmittel, die (a) zu einer sehr festen Haftung von Paclitaxel an der Ballonmembran führen und (b) in denen sich Wasser bei Raumtemperatur zu mindestens einem Volumenprozent löst. Aus diesen einfachen Lösungsmittelgemischen entstehen ohne technischen Aufwand Wirkstoffkristalle, im speziellen Fall Paclitaxelkristalle, die an dem gefalteten Ballon fest haften, sich bei Expansion des Ballons beispielsweise in einer verengten Arterie fast vollständig ablösen und zu einem hohen Anteil in das Gewebe übergehen. Dort erfolgt die Auflösung der Kristalle - wie aus der Pharmazie bekannt - langsam und sorgt damit über eine gewisse Zeit für eine wirksame Arzneimittelkonzentration. Die Aufbringung auf den Ballon ist mit dem oben beschriebenen Dosierverfahren wesentlich einfacher und im Hinblick auf die Dosis genauer als in der WO 2007/090385 beschrieben. Der Verzicht auf eine Matrix-Substanz hat den großen Vorteil, deren Kompatibilität mit dem Wirkstoff, die Langzeitstabilität der Matrix, Einfluß auf die Ballonmembran, und biologische Verträglichkeit nicht prüfen zu müssen. Hilfsstoffe können die Haftung von vormontierten Stents beeinflussen, beispielsweise indem die Haftung vermindert wird und der Stent vorzeitig verloren wird oder indem die Haftung verstärkt wird und sich der Stent nach Expansion nicht vom Ballon löst, was in beiden Fällen den Patienten gefährdet. Die Verminderung der Beladung der Ballone durch Verzicht auf Hilfsstoffe ist auch vorteilhaft, weil die zusätzlich aufgebrachte Substanz die notwendige enge Faltung der Ballone erschwert. Ein geringer Außendurchmesser der Ballone ist erforderlich, damit enge Stenosen passiert werden können.

Weitere erfindungsgemäße Ausführungsformen betreffen Katheterballon mit glattwandiger Ballonmembran mit einem Wirkstoff gelöst in einem organischen Lösungsmittel das mindestens 1%, bevorzugt mindestens 10% Wasser enthält, beschichtet, getrocknet und sterilisiert ist und wobei der Wirkstoff in kristalliner Form vorliegt. Dabei ist bevorzugt, wenn die Katheterballons mit glattwandiger Ballonmembran in gefaltetem Zustand beschichtet werden.

Eine andere bevorzugte Ausführungsform der vorliegenden Erfindung betrifft Ballonkatheter, bei denen die Ballonmembran, d.h. der Katheterballon des Katheterballons mit einem Wirkstoff gelöst in einem organischen Lösungsmittel das mindestens 1%, bevorzugt mindestens 10% Wasser enthält, beschichtet, getrocknet und sterilisiert ist und wobei der Wirkstoff auf der Ballonmembran in kristalliner Form vorliegt.

Den flüssigen Zubereitungen für die Beschichtung können wie bekannt lösliche, wasserlösliche oder mikropartikuläre Matrixsubstanzen beigefügt werden, wobei die partikuläre Matrixsubstanz auch der Wirkstoff selbst sein kann. Die Auswahl eines geeigneten Hilfsstoffs ist in den meisten Fällen vom Wirkstoff, dem Lösungsmittel und den Ballonoberflächen abhängig. Beispiele für geeignete, die Ablösung fördernde Hilfsstoffe sind Ascorbinsäure, Harnstoff und Polyethylenglycol bevorzugt in einem Molekulargewichtsbereich um 5000 bis 20000 D. Erfindungsgemäß wird Harnstoff als geeigneter, die Ablösung fördernder Hilfsstoff eingesetzt. Wegen des ungünstigen Einflusses der Beladung auf den Durchmesser und die Biegsamkeit der beschichteten Ballone sollte die Gesamtbeladung (Wirkstoff und Hilfsstoff) der Ballone, d.h. die insgesamt auf die Ballonmembran aufgetragene Dosis aller nicht flüchtigen Komponenten bevorzugt unter 10 µg/ mm², mehr bevorzugt unter 5 µg/mm² Ballonoberfläche (im expandierten Zustand) liegen, Hilfsstoffe sollen bevorzugt unter 1 µg/ mm² Ballonoberfläche dosiert werden, besonders bevorzugt unter 0,3 µg/ mm².

Die vorliegende Erfindung betrifft ferner einen Ballonkatheter, wobei die Ballonmembran des Katheterballons mit mindestens einem offen an deren Oberfläche aufliegenden Wirkstoff und Harnstoff in einer Weise beschichtet ist, dass der mindestens eine Wirkstoff bei der Expansion des Katheterballons sofort freigesetzt wird.

### Wasserlösliche und/oder hydrophile Wirkstoffe

Bisher werden zwei Klassen von Verbindungen zur lokalen Prophylaxe und Therapie arterieller Erkrankungen unterschieden: "...hydrophobic drugs, which are retained within tissue and have dramatic effects, and hydrophilic drugs, which are rapidly cleared and ineffective" (Levin AD, Vukmirovic N, Hwang C-W, Edelman ER. Specific binding to intracellular proteins determines arterial transport properties for rapamycin and paclitaxel. PNAS 2004;101:9463-9467). In US 6,306,166 werden ausdrücklich weitgehend wasserunlösliche Wirkstoffe für die Beschichtung gewählt. Beispielsweise hat sich die Beschichtung von Stents mit nicht ausreichend lipophilen Substanzen zur Restenoseprophylaxe als wirkungslos erwiesen (Muni NI et al. Am Heart J 2005;149:415-433; Kiesz RS et al. Circulation 2001;103:26-31; Kutryk MJB et al. J Am Coll Cardiol 2002;39:281-7). Huang y et al. Am J Cardiol berichten über eine wenn auch geringe Hemmung der Neointimaproliferation mit Stents, die mit Methotrexat beschichtet waren und den Wirkstoff aus einem Polymer langsam freisetzen. Die Verwendung von Methotrexat zur Beschichtung von Katheterballonen ist ungleich schwieriger, da sich der wasserlösliche Wirkstoff rasch auflöst, schon bevor der Ballon die Stenose im Blutgefäß erreicht hat. Auch für Arsentrioxid wurde eine Wirksamkeit nach langsamer Freisetzung aus einer Polymermatrix beschrieben (Yang W, Ge J, Liu H et al. Cardiovascular Research 2006;72:483-493). Die Begrenzung der Arzneistoffe auf lipophile Substanzen schränkt die Auswahl im Hinblick auf Wirksamkeit, Wirkprofil und Verfügbarkeit unerwünscht ein. Gerade unter den wasserlöslichen, weniger lipophilen Substanzen finden sich außerordentlich wirksame Arzneistoffe. Es wurde überraschend gefunden, dass wasserlösliche und/oder hydrophile Wirkstoffe trotz der gänzlich unterschiedlichen physikochemischen und pharmakokinetischen Eigenschaften bei einmaliger, nur kurze Zeit anhaltender Exposition von Zellen ebenso wie lipophile Arzneistoffe lang anhaltende Wirkungen verursachen können. Überraschenderweise besteht keine Notwendigkeit die rasche Verdünnung dieser Substanzen durch über lange Zeit anhaltende Freisetzung aus einem auf Dauer implantierten Reservoir zu kompensieren.

Bei der Verwendung hydrophiler, meist gut wasserlöslicher Arzneistoffe zur Verabreichung mittels beschichteter Medizinprodukte, speziell Ballonkatheter, treten schwierig zu lösende Probleme auf: Während die schlecht wasserlöslichen Substanzen wie Paclitaxel oder Rapamycin und dessen Derivate in Einführungsschleusen, Führungskathetern und im Blut an der Oberfläche der beschichteten Medizinprodukte zum großen Teil haften bleiben und sich erst bei mechanischer Belastung, z.B. beim Expandieren eines Ballons und dessen Reibung an der Gefäßwand ablösen und ggf. in Gegenwart von Proteinen und Membranlipiden auflösen, lösen sich hydrophile Substanzen meist beim ersten Kontakt mit Wasser bzw. Blut und gehen so zum großen Teil vor Erreichen des Zielorts verloren. Hydrophile Wirkstoffe benötigen daher in der Regel Schutzmaßnahmen zur Verhinderung der Freisetzung beim Gebrauch während der kurzen Zeit zwischen dem ersten Kontakt des sterilen beschichteten Medizinproduktes mit wässrigen Flüssigkeiten, beispielsweise Blut, vor Erreichen des Zielortes und dem Erreichen des Zielortes. Diese Maßnahmen sind nicht zu verwechseln mit Formulierungen, die eine verzögerte Freigabe des Wirkstoffs am Zielort bewirken, um eine lang anhaltende Wirkung zu gewährleisten. Die Freisetzung der hydrophilen, wasserlöslichen Wirkstoffe soll sofort erfolgen, wenn das Medizinprodukt den Wirkort erreicht hat, jedoch nicht vorher.

Eine überraschende Besonderheit weist Arsentrioxid trotz seines hydrophilen Charakters auf. Es kann als Lösung auf die Ballonoberfläche aufgetragen werden, haftet nach dem Trocknen jedoch fest an der Membran und wird bei Expansion der Ballone annähernd vollständig abgegeben.

Das Problem mit hydrophilen und/oder wasserlöslichen Wirkstoffen beginnt mit dem Auftragen auf die Oberfläche der Medizinprodukte. Viele dieser Oberflächen, insbesondere die gebräuchlichen Katheter lassen sich mit wässrigen oder anderen hydrophilen Lösungsmitteln nicht oder nur sehr ungleichmäßig benetzen. Eine weitere wesentliche Eigenschaft ist die Haftung der Beschichtung an der Oberfläche des Medizinproduktes oder - spezieller - der Ballonmembran. Gleichmäßigkeit der Verteilung der Beschichtung und Haftungseigenschaften lassen sich durch geringe Modifizierung der Oberflächen überraschend deutlich beeinflussen. So haben mit aktiviertem Sauerstoff ('Plasma') behandelte Oberflächen nicht nur eine gleichmäßigere Verteilung sondern insbesondere auch eine sehr gute Haftung an den gefalteten Membranen und Ablösung der Beschichtung bei Expansion von Ballonen gezeigt. Ähnliche Ergebnisse werden mit hydrophil derivatisierten oder beschichteten Membranen erzielt.

Als Lösungsmittel zum Auftrag von hydrophilen und/oder wasserlöslichen Wirkstoffen ist Wasser nur bedingt geeignet. Bevorzugt sind mit Wasser mischbare, relativ hydrophile organische Lösungsmittel wie Methanol, Ethanol, Propanol, Isopropanol, Dimethylsulfoxid, Aceton, Ameisensäure, Essigsäure, Ammoniak, Tetrahydrofuran, Dimethylformamid, Dimethylacetamid etc., deren Gemische untereinander und mit Wasser, wobei der pH der Lösung mit Säuren oder Basen angepasst werden kann. Die Lösungsmittel werden soweit möglich und erwünscht vor Gebrauch der Medizinprodukte abgedampft, ggf. unter Einwirkung erhöhter Temperaturen und vermindertem Druck.

Die hydrophilen und/oder wasserlöslichen Stoffe können als solche oder als Salze gelöst werden. Im Falle der Anthracycline, speziell des Doxorubicins können durch geeignete Wahl der Konzentration und der Ionenkonzentration, bevorzugt der Natriumionenkonzentration und des pH viskose Lösungen in Wasser hergestellt werden (Hayakawa E, Furuya K, Kuroda T, Moriyama M, Kondo A. Viscosity study on the self-association of doxorubicin in aqueous solution. Chem. Pharm Bull 1991;39:1282-1286), die sich überraschend gut zur Beschichtung von Oberflächen eignen. Obwohl diese Lösungen ausschließlich Wasser als Lösungsmittel enthalten können, lassen sich sehr gleichmäßige Beschichtungen erzielen, selbst wenn die Membranen eher lipophil sind wie bei gebräuchlichen Ballonkathetern.

Alle oben genannten Beschichtungen werden nach einem der üblichen Verfahren durch Tauchen, Sprühen, Bestreichen oder mittels einer Volumenmeßeinrichtung aufgetragen, bevorzugt nach dem oben beschriebenen Verfahren mit einer Volumendosiereinrichtung. Im Falle von Ballonkathetern können die Ballone im expandierten oder gefalteten oder intermediären Zustand beschichtet werden.

Eine weitere Möglichkeit der Beschichtung mit hydrophilen und/oder wasserlöslichen Stoffen ergibt sich dadurch, dass die Substanzen nicht in gelöstem Zustand auf die Oberflächen aufgebracht werden. Die hydrophilen und/oder wasserlöslichen Stoffe können beispielsweise als Festsubstanz, in Form von Mikro- oder Nanopartikeln in Flüssigkeiten eingebracht werden, in denen sie nur wenig löslich sind oder sie werden aus Flüssigkeiten in denen sie löslich sind ausgefällt. Dies erlaubt die Verwendung lipophiler organischer Lösungsmittel und den Zusatz lipophiler Hilfsstoffe in Verbindung mit hydrophilen und/oder wasserlöslichen Stoffen. Durch die Beschichtung von Oberflächen mit vorgeformten Partikeln und ggf. dem Zusatz lipophiler Hilfsstoffe in lipophilen Lösungsmitteln wird einer vorzeitigen Ablösung der Beschichtung vorgebeugt.

Viele hydrophile und/oder wasserlösliche Stoffe enthalten funktionelle Gruppen, die elektrisch geladen sein können. Sie können in elektrisch ungeladenem Zustand in organischen Lösungsmitteln löslich sein und in dieser Form zur Beschichtung verwendet werden. Sie können gut lösliche oder schwer lösliche Salze bilden. Eine bevorzugte Möglichkeit der Beschichtung von Medizinprodukten ist die Verwendung schwer löslicher Salze hydrophiler und/oder wasserlöslicher Stoffe. Dadurch wird einer vorzeitigen Ablösung nach Kontakt mit beispielsweise physiologischen Lösungen wie sie zur Benetzung von Kathetern verwendet werden oder mit Blut in Einführschleusen, Führungskathetern oder direkt im Blutstrom vermieden. Durch die Bildung eines unlöslichen Salzes wird die Wirksamkeit von Arzneimitteln nicht aufgehoben. Das schwer lösliche Salz setzt den unveränderten Arzneistoff nach Ablösung von dem Medizinprodukt wieder frei, was bei den außerordentlich geringen Arzneistoffmengen, die bei lokaler Verabreichung für eine gute Wirksamkeit notwendig sind, vollständig genügt. Das gleiche Prinzip kann für hydrophile, an sich wasserlösliche Hilfsstoffe verwendet werden. Die Umwandlung in ein schwer lösliches Salz schafft eine schwer lösliche Matrix-Struktur, die einen hydrophilen und/oder wasserlöslichen Wirkstoff über einige Zeit, z.B. während der Manipulation eines Ballonkatheters vor der eigentlichen Gefäßdilatation, vor der vorzeitigen Ablösung schützt.
Die unlöslichen Salze können vor der Verwendung der hydrophilen und/oder wasserlöslichen Stoffe zur Beschichtung der Medizinprodukte hergestellt und dann in Form von Suspensionen in geeigneten Trägerflüssigkeiten eingesetzt werden. Ein bevorzugter Weg ist die Beschichtung der Medizinprodukte mit der löslichen Form in wässriger Lösung oder wasserhaltigem organischen Lösungsmittel oder relativ hydrophilem organischen Lösungsmittel oder Lösungsmittelgemisch, dem Abdampfen des Lösungsmittels und der anschließenden Behandlung der so beschichteten Oberfläche mit einem Fällungsmittel für die hydrophilen und/oder wasserlöslichen Stoffe und damit die nachträgliche Umwandlung in das unlösliche Salz oder die unlösliche, elektrisch nicht geladene Form. Das Fällungsmittel kann in beliebiger Form, z.B. durch Tauchen, sprühen, Bestreichen oder mit einer Volumenmeßeinrichtung aufgetragen werden.

Beispiele für physiologisch akzeptable schwer lösliche Salze sind Calcium-, Magnesium-, Zink- und Eisen II- oder Eisen III- Verbindungen einerseits und Phosphate, Sulfate, Oxalate oder auch Salze ionischer Röntgenkontrastmittel wie Diatrizoate etc. andererseits.
Somit betrifft die vorliegende Erfindung auch die Verwendung mindestens eines hydrophilen niedermolekularen Wirkstoffs in Form eines schlecht wasserlöslichen Salzes oder als schlecht wasserlösliche Säure oder schlecht wasserlösliche Base zur Behandlung und Prophylaxe von Gefäßerkrankungen als auch zur Erzielung anhaltender Wirkungen nach einmaliger Verabreichung bei sofortiger Bioverfügbarkeit.
Bei einer weiteren erfindungsgemäßen Ausführungsform ist die Ballonmembran des Katheterballons mit mindestens einem offen an deren Oberfläche aufliegenden Wirkstoff und Harnstoff in einer Weise beschichtet, dass der mindestens eine Wirkstoff bei der Expansion des Katheterballons sofort freigesetzt wird, wobei der mindestens eine an sich wasserlösliche Wirkstoff als schlecht wasserlösliches Salz oder als schlecht wasserlösliche Säure oder schlecht wasserlösliche Base oder schlecht wasserlösliche Komplexverbindung vorliegt.
Eine ähnliche erfindungsgemäße Ausführungsform betrifft eine Ballonmembran des Katheterballons mit mindestens einem offen an deren Oberfläche aufliegenden Wirkstoff und Harnstoff, die in einer Weise beschichtet ist, dass der mindestens eine Wirkstoff bei der Expansion des Katheterballons sofort freigesetzt wird, wobei der mindestens eine Wirkstoff nach dem Auftragen auf die Ballonmembran oder die hydrophile Ballonmembran oder die hydrophil beschichtete Ballonmembran in eine schlecht wasserlösliche Form insbesondere ein schlecht wasserlösliches Salz oder eine schlecht wasserlösliche Säure oder eine schlecht wasserlösliche Base oder eine schlecht wasserlösliche Komplexverbindung überführt worden ist.
Der Verlust hydrophiler und/oder wasserlöslicher Wirkstoffe von Medizinprodukten während der Handhabung, insbesondere auf dem Weg durch Einführschleusen oder Führungskatheter zum Ort der Behandlung kann weiterhin durch die nachträgliche Beschichtung mit wenig oder langsam wasserlöslichen physiologisch annehmbaren Substanzen erreicht werden. Dabei können diese Substanzen eine erwünschte pharmakologische Wirkung haben oder als Hilfsstoffe dienen. Die Überzüge können fest oder wie im Falle von bestimmten Lipiden auch flüssig sein. Beispiele für feste Überzüge sind Zucker, Zuckeralkohole, andere organische Neutralstoffe, lipophile Aminosäuren, Salze organischer und anorganischer Säuren und Basen, in der Medizin gebräuchliche Kontrastmittel oder Farbstoffe, Gerinnungshemmer wie Heparin, Plättchenaggregationshemmer wie Acetylsalicylsäure, Salicylsäure und viele andere. Es ist im Einzelfall zu prüfen, wie wirksam der Schutz einer Beschichtung durch einen speziellen Überzug ist. Schützende Überzüge werden bevorzugt dadurch aufgebracht, dass Lösungen in Lösungsmitteln verwendet werden, in denen die zu schützende Beschichtung nicht löslich ist. Beispielsweise ist Acetylsalicylsäure (als Schutzüberzug) gut in Ethylacetat löslich, in dem viele der hydrophilen und/oder wasserlöslichen Wirkstoffe sehr wenig löslich sind.
Schutzüberzüge sollen möglichst dünn sein. Bevorzugt ist ein Auftrag von < 30 µg/mm² Oberfläche.
Schutzüberzüge können wiederum auf unterschiedliche Weise aufgetragen werden, wobei Sprühen und sehr kurzzeitiges Tauchen bevorzugt sind.
Somit betrifft die vorliegende Erfindung in einer weiteren bevorzugten Ausführungsform Ballonkatheter, die mit mindestens einem hydrophilen Wirkstoff und Harnstoff oder einer mindestens einen hydrophilen Wirkstoff und Harnstoff enthaltenden Zubereitung beschichtet sind, wobei auf diese Schicht eine weitere äußere Schutzschicht in Form eines wenig oder langsam wasserlöslichen biokompatiblen Materials aufgetragen worden ist. Somit sind Ballonkatheter bevorzugt, bei denen der mindestens eine Wirkstoff oder der mindestens eine hydrophile Wirkstoff mit einer wenig oder langsam wasserlöslichen biokompatiblen Schicht überzogen oder durchtränkt ist. Die Schutzschicht kann die Wirkstoffschicht durchdringen. Sie kann beispielsweise beispielsweise aus biologisch unwirksamen Substanzen, aber auch aus Acetylsalicylsäure oder Heparin bestehen.

### Beispiele

### Referenzbeispiel 1

### Beschichtung von hydrophilen und nicht hydrophilen Ballonkathetern mit Paclitaxel Versuche Nr. 102/103 und 128/129

Beschichtungslösung: 30 mg Paclitaxel/ml in Aceton 89%, Ethanol 9%, Ultravist® - 370 (Schering AG, Berlin) 2 % durch 4maliges Tauchen mit zwischenzeitlichem Trocknen:

| Art der Katheter | Zahl der Ballonkatheter | µg Paclitaxel/ mm² | |
|---|---|---|---|
| | | Ballonoberfläche | Standardabweichung |
| Standard, 3,5 -15 mm | 5 | 3.4 | 0.5 |
| Hydrophil, 3,5 -15 mm | 5 | 2.8 | 0.2 |
| Standard, 3,5-20 mm | 8 | 5.0 | 0.5 |
| Hydrophil 3,5-20 mm | 8 | 5.6 | 0.2 |

Schlussfolgerung: Ballone mit hydrophiler Oberfläche sind reproduzierbarer zu beschichten.

### Referenzbeispiel 2

### Beschichtung mit einer Mikrodosiereinrichtung, Vergleich zum Beschichten durch Tauchen

In der ersten Serie (Versuch Nr. 323 und 326-329) wurden je 3 fertig gefaltete Ballone entweder 4 mal in die Beschichtungslösung A getaucht und zwischen den Tauchvorgängen jeweils gründlich getrocknet oder der Wirkstoff wurde mit einer Hamilton CR-700 Constant Rate Kolben-Spritze 2 mal mit je 12,5 µl der gleichen Lösung oder 3 weiteren Beschichtungslösungen in leicht flüchtigen organischen Lösungsmitteln aufgetragen.

| **Versuch Nr. (n)** | **Ballon** | **Ballon-Zustand** | **Lösungsmittel** | **WirkstoffKonzentration** | **Zahl der Tauchbeschichtungen** | **Wirkstoff auf dem Balloon µg/mm², Mittel ± SD** |
|---|---|---|---|---|---|---|
| 323 (3) | 3,0-17 | gefaltet | A. | 30 mg/ml | **4** | 4,1±0,7 |

| | | | | | **Zahl der Dosiervorgänge und Volumen** | |
|---|---|---|---|---|---|---|
| 326 (3) | 3,0-17 | gefaltet | A | 30 mg/ml | 2 x 12,5 µl | 3,6±0,0 |
| 327 (3) | 3,0-17 | gefaltet | B | 30 mg/ml | 2 x 12,5 µl | 3,6±0,1 |
| 328 (3) | 3,0-17 | gefaltet | C | 30 mg/ml | 2 x 12,5 µl | 3,8±0,1 |
| 329 (3) | 3,0-17 | gefaltet | D | 30 mg/ml | 2 x 12,5 µl | 3,7±0,0 |

Die Dosierung mit der Hamilton-Spritze führte zu einer deutlich genaueren Dosierung auf den Ballonen.

Die Einhaltung der Dosis gelang auch auf Ballonen unterschiedlicher Größe (Versuch 390/ 391) und war genauer als bei Verwendung der Tauchbeschichtung (392).

| **Versuch Nr. (n)** | **Ballon** | **Ballon-Zustand** | **Lösungsmittel** | **WirkstoffKonzentration** | **Zahl der Dosiervorgänge und Volumen** | **Wirkstoff auf dem Balloon µg/mm², Mittel ± SD** |
|---|---|---|---|---|---|---|
| 390(4) | 3,5-20 | gefaltet | A | 30 mg/ml | 2x14 µl | 3,7±0,1 |
| 391 (4) | 2,0-14 | gefaltet | A | 30 mg/ml | 2x6 µl | 3,6±0,1 |
| | | | | | **Zahl der Tauchbeschichtungen** | |
| 392 (4) | 3,5-20 | gefaltet | A | 30 mg/ml | 4 | 4,4±0,4 |

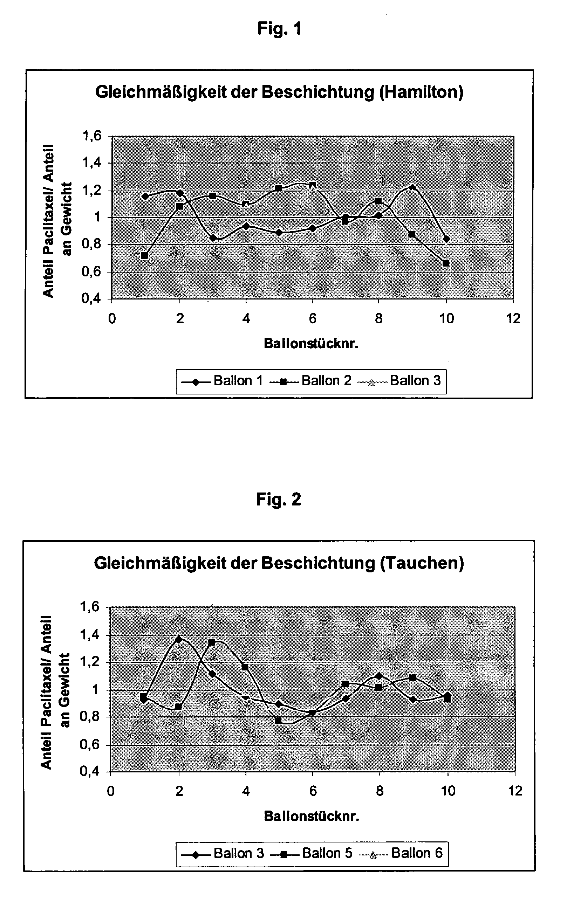

Die Verteilung des Wirkstoffs entlang der Längsachse der Ballone wurde am Beispiel von je 3 Stück 100 mm langen PTA-Ballonen mit 5 mm Durchmesser untersucht. Die Ballone wurden nach dem Beschichten mit entweder dem Dosierverfahren mit Hamilton-Spritze (s. Fig. 1) oder dem Tauchen (s. Fig. 2) in 10 mm lange Teilstücke geschnitten. Der Wirkstoffgehalt der Teilstücke wurde mittels HPLC gemessen. In der Abbildung bedeutet ein Y-Achsenabschnitt von 1 die Durchschnittsmenge über die gesamte Länge, d.h. die ideale Gleichverteilung.

Die Verteilung des Wirkstoffs auf der Längsachse der Ballone ist nach Auftragen mit dem Dosierverfahren keinesfalls ungleichmäßiger, eher gleichmäßiger als nach dem Tauchen der Ballone in die Lösung (s. Fig. 1 und 2).

### Referenzbeispiel 3

### Überleitung zum Aufbringen der Beschichtungslösung auf den Ballon:

Bevorzugt wird eine englumige Nadel 2 - 10 cm lang, proximal mit Anschluß an die Mikrodosiereinrichtung, distal endständig geschlossen. Die Nadel hat einen seitlichen Auslaß in Form einer gerundeten Kerbe, der sich der Rundung des Ballons anpasst (s. Fig. 3).

### Referenzbeispiel 4

### Beschichtung von Ballonkathetern mit Methotrexat

### Beschichtungslösung:

30 mg Methotrexinsäure + 100 µl Sodiumbicarbonat (7,5 %) + 900 µl Methanol (Beschichtung mit 2x16 µl, entspr. ~ 4 µg/mm² Ballonoberfläche)

| | |
|---|---|
| Ballone: | 3,5 -19 mm |
| Überzug: | Ultravist®-370 + Hydroxyethylstärke (HAES) 10 % (1/1 Vol) + 30 mg Methotrexat/ml aufgetragen durch kurzzeitiges Tauchen |
| Stents: | Edelstahl, ballonexpandierbar, 3.5 - 18 mm |
| Wirkstoffgehalt: | 5,3 µg / mm² Ballonoberfläche |

### Referenzbeispiel 5

### Wirksamkeit und Verträglichkeit der beschichteten Ballonkatheter nach Beispiel 5 am Schwein in überdehnten Koronararterien

Methode: Scheller B, Speck U, Abramjuk C, Bernhardt U, Böhm M, Nickenig G: Paclitaxel balloon coating - a novel method for prevention and therapy of restenosis. Circulation 2004; 110: 810-4
Den Schweinen wurden Stents mit Hilfe der Methotrexat-beschichteten oder mit unbeschichteten (Kontrolle) Ballonkathetern implantiert. Nach 4 Wochen wurde mit Hilfe der quantitativen Angiographie das Ausmaß der Verengung des Lumens im Bereich des Stents gemessen.

### Ergebnis:

| | **Kontrolle n=9** | **Methotrexate n=8** | **p** |
|---|---|---|---|
| Referenzdurchmesser [mm] | 2,41 ± 0,28 | 2,30 ± 0,39 | 0,495 |
| Stentdurchmesser [mm] | 2,64 ± 0,13 | 2,41 ± 0,29 | 0,045 |
| Überdehnungsrate [-] | 1,11 ± 0,14 | 1,07 ± 0,20 | 0,068 |
| Referenzdurchmesser 28d [mm] | 2,35 ± 0,39 | 2,38 ± 0,26 | 0,840 |
| Minimaler Gefäßdurchmesser 28d [mm] | 1,54 ± 0,32 | 1,74 ± 0,41 | 0,273 |
| Late lumen loss [mm] | 1,10 ± 0,33 | 0,67 ± 0,39 | 0,025 |

Der "late lumen loss" bedeutet, dass von dem ursprünglichen Durchmesser des mit Blut durchströmten Lumens der Koronararterien von 2.64 bzw. 2.41 mm in der Kontrollgruppe (kein Methotrexat) 1,1 mm und in der mit Methotrexat behandelten Gruppe 0,67 mm innerhalb von 4 Wochen durch exzessives Zellwachstum verloren ging. Methotrexat hat folglich die unerwünschte, das Gefäßlumen einengende Proliferation der Arterienwand signifikant (p< 0,025) vermindert.

### Referenzbeispiel 6

### Beschichtung von Ballonkathetern mit Thalidomid

Falcon Bravo RX 3.5 - 20 mm, Invatec S.R.L., Roncadelle, Italien 8 Stück; Beschichtungslösung:
Dimethylformamid + 50mg/ml Thalidomid
Beschichtung je Ballon 2 mal 8 µl, nach jedem Beschichten mindestens 12h trocknen;
danach 4 Ballone kurz in 50 mg Trimyristine in 3 ml warmen Ethylacetat getaucht. Verlust durch Einführschleuse, Führungskatheter und 1 min in einer Koronararterie des Schweins (nicht expandiert) und zurückgezogen. Analyse mit HPLC, Säule Waters Symmetry, C18, 5µm, 25cmx4,6mm, mobile Phase: 72 vol% 0,01 M Ammoniumacetatpuffer, pH 5,5, und 28 vol% aus Acetonitril, 0.8 ml/min, Detektion 300 nm.

Die mit Trimyristat behandelten Ballone verloren im Mittel 28% des Wirkstoffs auf dem Weg in die Koronarie und zurück, die nicht mit Trimyristat behandelten Ballone 95 %, d.h. die Trimyristat-Beschichtung hat die Haftung von Thalidomid am Ballon deutlich verbessert.

### Referenzbeispiel 7

### Beschichtung von Ballonkathetern mit Arsentrioxid

Falcon Bravo RX 3.5 - 20 mm, Invatec S.R.L., Rocadelle, Italien 12 Stück Beschichtungslösung:
50 mg As₂O₃ werden in 1ml Wasser für Injektionszwecke gelöst, die Lösung wird mit 3 ml Aceton oder Methanol verdünnt.
Beschichtung je Ballon 3 mal 25 µl, nach jedem Beschichten mindestens 12h trocknen;
Verlust durch Einführschleuse, Führungskatheter und 1 min in einer Koronararterie des Schweins (nicht expandiert) und zurückgezogen bzw. nach Expansion in einer Koronararterie für 1 min.; Analyse nach Veraschen mittels Atomabsorptionsspektrometrie.

Die Ballone verloren im Mittel 25% des Wirkstoffs auf dem Weg in die Koronarie und zurück, nach Expansion in der Arterie verblieben im Mittel 13% der Dosis auf den Ballonen (jeweils n=4).

### Referenzbeispiel 8a

### Steuerung der Haftung allein durch das Lösungsmittel (Serie 1)

Falcon Bravo RX 3.5 - 20 mm, Invatec S.R.L., Rocadelle, Italien 12 Stück

Die Ballone wurden in gefaltetem Zustand mit je 3 - 4 µg/mm² Paclitaxel beschichtet und wie unter 'Definitionen' angegeben auf Verlust des Wirkstoffs während der Expansion in trockenem Zustand geprüft:

| | |
|---|---|
| Aceton | 21% |
| Dioxan | 12% |
| Dimethylformamid | 24% |
| Dimethylsulfoxid | 66% |
| Essigsäure | 4% |
| Isopropanol | 19% |
| Tetrahydrofuran | 4% |

### Referenzbeispiel 8b

### Steuerung der Haftung allein durch den Zusatz von Wasser bis zur Löslichkeitsgrenze Wasser in Tetrahydrofuran (THF) bei Raumtemperatur (Serie 2)

| | |
|---|---|
| Tetrahydrofuran | 3% |
| Tetrahydrofuran mit 10 Vol% Wasser | 3% |
| Tetrahydrofuran mit 20 Vol % Wasser | 16% |
| Tetrahydrofuran mit 37.5 Vol% Wasser | 37% |

### Beispiel I

### Beschichtung eines Ballonkatheters mit Zusatz von Harnstoff

Falcon Bravo RX 3.5 - 20 mm, Invatec S.R.L., Roncadelle, Italien 8 Stück; Beschichtungslösung:
70 mg Harnstoff gelöst in 1 ml Wasser + 9 ml Tetrahydrofuran + 500 mg Paclitaxel Beschichtung je Ballon 1 mal 18 µl mit der Mikrodosiereinheit nach Beispiel 2.

## Patentansprüche

1. Ballonkatheter umfassend einen Katheterballon mit einer Ballonmembran, die hydrophil oder hydrophilisiert ist und/oder die Oberfläche der Ballonmembran eine hydrophile Beschichtung trägt, wobei die Ballonmembran dafür geeignet ist mit einer Gefäßwand in Berührung zu kommen, wobei die Ballonmembran mit mindestens einem offen an deren Oberfläche aufliegenden Wirkstoff und Harnstoff beschichtet ist und wobei der mindestens eine Wirkstoff bei der Expansion des Katheterballons sofort freigesetzt wird.

2. Ballonkatheter nach Anspruch 1, wobei die Ballonmembran des Katheterballons mit aktiviertem Sauerstoff behandelt ist.

3. Ballonkatheter nach einem der Ansprüche 1 - 2, wobei sich der Radius des Katheterballons nach vollständiger Entfaltung durch Druckerhöhung im Inneren des Katheterballons um mehr als 15 % vergrößert.

4. Ballonkatheter nach einem der Ansprüche 1 - 3, wobei der Katheterballon einen Berstdruck von unter 10.000 hPa hat.

5. Ballonkatheter nach einem der Ansprüche 1 - 4, wobei der Katheterballon nach vollständiger Entfaltung einen gegenüber dem Referenzdurchmesser der Arterie um mindestens 20% größeren Durchmesser besitzt.

6. Ballonkatheter nach einem der Ansprüche 1 - 5, wobei die Ballonmembran des Katheterballons mit mindestens einem hydrophilen Wirkstoff beschichtet worden ist.

7. Ballonkatheter nach einem der Ansprüche 1 - 6, wobei die Ballonmembran des Katheterballons mit mindestens einem offen an deren Oberfläche aufliegenden Wirkstoff in einer Weise beschichtet ist, dass der mindestens eine Wirkstoff bei der Expansion des Katheterballons sofort freigesetzt wird, wobei der mindestens eine an sich wasserlösliche Wirkstoff als schlecht wasserlösliches Salz oder als schlecht wasserlösliche Säure oder schlecht wasserlösliche Base oder schlecht wasserlösliche Komplexverbindung vorliegt.

8. Ballonkatheter nach einem der Ansprüche 1 - 7, wobei die Ballonmembran des Katheterballons mit mindestens einem offen an deren Oberfläche aufliegenden Wirkstoff in einer Weise beschichtet ist, dass der mindestens eine Wirkstoff bei der Expansion des Katheterballons sofort freigesetzt wird, wobei der mindestens eine Wirkstoff nach dem Auftragen auf die Ballonmembran in eine schlecht wasserlösliche Form überführt worden ist.

9. Ballonkatheter nach einem der Ansprüche 1 - 8, wobei der mindestens eine Wirkstoff oder der mindestens eine hydrophile Wirkstoff mit einer wenig oder langsam wasserlöslichen biokompatiblen Schicht überzogen oder durchtränkt ist.

10. Ballonkatheter nach einem der Ansprüche 1 - 9, wobei der Katheterballon beliebige Schutzüberzüge enthält.

11. Ballonkatheter nach einem der Ansprüche 1 - 10, wobei der Katheterballon mit einer Zubereitung beschichtet wurde, die mindestens ein leicht flüchtiges Lösungsmittel oder mindestens eine Chlorverbindung oder Fluorverbindung mit einem Siedepunkt unter 300°C enthält.

12. Ballonkatheter nach einem der Ansprüche 1 - 11, wobei der Katheterballon mit einem Gel beschichtet ist, wobei der mindestens eine Wirkstoff selbst als Gelbildner fungiert oder an der Gelbildung teilnimmt.

13. Ballonkatheter nach einem der Ansprüche 1 - 12, wobei die Beschichtungszusammensetzung mindestens ein hydrophiles Lösungsmittel oder ein Gemisch mindestens eines hydrophilen Lösungsmittels mit Wasser enthält.

14. Ballonkatheter nach einem der Ansprüche 1 - 13, wobei die Ballonmembran des Katheterballons mit einem Wirkstoff gelöst in einem organischen Lösungsmittel das mindestens 1% Wasser enthält, beschichtet, getrocknet und sterilisiert ist und wobei der Wirkstoff auf der Ballonmembran in kristalliner Form vorliegt.

15. Ballonkatheter nach einem der Ansprüche 1 - 14, wobei der Katheterballon mit glattwandiger Ballonmembran mit einem Wirkstoff gelöst in einem organischen Lösungsmittel das mindestens 1% Wasser enthält, beschichtet, getrocknet und sterilisiert ist und wobei der Wirkstoff in kristalliner Form vorliegt.

16. Ballonkatheter nach Anspruch 15, wobei der Katheterballon mit glattwandiger Ballonmembran in gefaltetem Zustand beschichtet ist.

17. Ballonltatheter nach einem der Ansprüche 13 bis 16, wobei das organische Lösungsmittel Methanol, Ethanol, Propanol, Iso-Propanol, Aceton, Tetrahydrofuran, Essigsäure, Dioxan oder Dimethylformamid enthält.

18. Ballonkatheter nach einem der Ansprüche 1 bis 17, wobei die insgesamt auf die Ballonmembran aufgetragene Dosis aller nicht flüchtigen Komponenten unter 10 µg/mm² liegt.

19. Ballonkatheter nach einem der Ansprüche 1 - 18, enthaltend Methotrexinsäure Arsen oder Arsenverbindungen, Wismuth oder Wismuthverbindungen, oder Thalidomid.

20. Ballonkatheter nach einem der Ansprüche 1 - 19 geeignet zur lokalen Behandlung und Prophylaxe von Gefäßerkrankungen, und/oder zur Behandlung von Gefäßwandveränderungen, die den Blutfluss nicht wesentlich einschränken.

21. Verfahren zur Herstellung von Katheterballons eines Ballonkatheters nach einem der Ansprüche 1-20 umfassend die Schritte:
a) Bereitstellung eines Katheterballons mit einer Ballonmembran, die hydrophil oder hydrophilisiert ist und/oder die Oberfläche der Ballonmembran eine hydrophile Beschichtung trägt,
b) Bereitstellung einer Mikrodosiereinheit enthaltend eine Beschichtungszusammensetzung die nicht im Kontakt mit einer Gasphase steht, wobei der Beschichtungszusammensetzung einen Wirkstoff und Harnstoff umfasst,
c) verlustfreie und gleichmäßige Beschichtung des Katheterballons mit der Beschichtungszusammensetzung unter Verwendung der Mikrodosiereinheit.

22. Verfahren nach Anspruch 21, wobei sich der Katheterballon während der Beschichtung um seine Längsachse dreht und sich die Mikrodosiereinheit entlang der Längsachse des Katheterballons hin und her bewegt.

## Claims

1. A balloon catheter comprising a catheter balloon with a balloon membrane, which is hydrophilic or hydrophilised and/or the surface of the balloon membrane bears a hydrophilic coating, wherein the balloon membrane is suitable for coming into contact with a vessel wall, wherein the balloon membrane is coated with at least one active substance openly applied on its surface and urea, and wherein the at least one active substance is immediately released on expansion of the catheter balloon.

2. The balloon catheter according to claim 1, wherein the balloon membrane of the catheter balloon is treated with activated oxygen.

3. The balloon catheter according to any one of claims 1 - 2, wherein after complete expansion through increasing the pressure on the inside of the catheter balloon, the radius of the catheter balloon increases by more than 15 %.

4. The balloon catheter according to any one of claims 1 - 3 wherein the catheter balloon has a burst pressure of under 10,000 hPa.

5. The balloon catheter according to any one of claims 1 - 4 wherein after complete expansion the balloon catheter has a diameter at least 20 % greater than the reference diameter of the artery.

6. The balloon catheter according to any one of claims 1 - 5, wherein the balloon membrane of the catheter balloon has been coated with at least one hydrophilic active substance.

7. The balloon catheter according to any one of claims 1 - 6, wherein the balloon membrane of the catheter balloon is coated with at least one active substance openly applied to its surface in such a way that the at least one active substance is immediately released upon expansion of the catheter balloon, wherein the at least one it itself water-soluble active substance is present in the form of a salt that is poorly water-soluble or an acid that is poorly water-soluble or a base that is poorly water-soluble or a complex compound that is poorly water-soluble.

8. The balloon catheter according to any one of claims 1 - 7, wherein the balloon membrane of the catheter balloon is coated with at least one active substance openly applied to its surface in such a way that the at least one active substance is immediately released upon expansion of the catheter balloon, wherein after application onto the balloon catheter the at least one active substance is converted into a form that is poorly soluble in water.

9. The balloon catheter according to any one of claims 1 - 8, wherein the at least one active substance or the at least one hydrophilic substance is coated or impregnated with a biocompatible layer that is poorly or slowly soluble in water.

10. The balloon catheter according to any one of claims 1 - 9, wherein the catheter balloon contains any number of protective coatings.

11. The balloon catheter according to any one of claims 1 - 10, wherein the catheter balloon has been coated with a preparation which contains at least one an easily volatile solvent or at least one chlorine compound or fluorine compound with a boiling point of below 300 °C.

12. The balloon catheter according to any one of claims 1 - 11, wherein the catheter balloon is coated with a gel wherein the at least one active substance itself acts as a gelling agent or takes part in the gel formation.

13. The balloon catheter according to any one of claims 1 - 12, wherein the coating composition contains at least one hydrophilic solvent or a mixture of at least one hydrophilic solvent with water.

14. The balloon catheter according to any one of claims 1 - 13, wherein the balloon membrane of the catheter balloon is coated with an active substance dissolved in an organic solvent containing at least 1 % water, is dried and sterilised, and wherein the active substance is present on the balloon membrane in crystalline form.

15. The balloon catheter according to any one of claims 1 - 14, wherein the catheter balloon with smooth-walled balloon membrane is coated with an active substance dissolved in an organic solvent containing at least 1 % water, is dried and sterilised, and wherein the active substance is present in crystalline form.

16. The balloon catheter according to claim 15, wherein the balloon catheter with smooth-walled balloon membrane is coated in a folded state.

17. The balloon catheter according to any one of claims 13 to 16, wherein the organic solvent contains methanol, ethanol, propanol, isopropanol, acetone, tetrahydrofurane, acetic acid, dioxane or dimethyl formamide.

18. The balloon catheter according to any one of claims 1 to 17, wherein the dose of all non-volatile components applied to the balloon membrane is below 10 µg/mm².

19. The balloon catheter according to any one of claims 1 to 18, containing methotrexin acid, arsenic or arsenic compounds, bismuth or bismuth compounds or thalidomide.

20. The balloon catheter according to any one of claims 1 - 19 suitable for the local treatment and prophylaxis of vascular diseases and/or for the treatment of vascular wall changes which do not essentially restrict the blood flow.

21. A method of manufacturing catheter balloons of a balloon catheter according to any one of claims 1 - 20 comprising the steps:
a) provision of a catheter balloon with a balloon membrane that is hydrophilic or hydrophilised and/or the surface of the balloon membrane bears a hydrophilic coating,
b) provision of a micro-dosing unit comprising a coating composition which is not in contact with a gaseous phase, wherein the coating composition contains an active substance and urea,
c) loss-free and even coating of the catheter balloon with the coating composing using the micro-dosing unit.

22. The method according to claim 21, wherein during the coating the catheter balloon is turned about its longitudinal axis and the micro-dosing unit moves back and forth along the longitudinal axis of the catheter balloon.

## Revendications

1. Cathéter à ballonnet comprenant un ballonnet de cathéter présentant une membrane de ballonnet qui est hydrophile ou rendue hydrophile et/ou la surface de la membrane de ballonnet porte un revêtement hydrophile, dans lequel la membrane de ballonnet est appropriée pour entrer en contact avec une paroi vasculaire, dans lequel la membrane de ballonnet est revêtue avec au moins une substance active et de l'urée appliquées de façon ouverte sur sa surface et dans lequel l'au moins une substance active est libérée immédiatement lors de l'expansion du ballonnet de cathéter.

2. Cathéter à ballonnet selon la revendication 1, dans lequel la membrane de ballonnet du ballonnet de cathéter est traitée avec de l'oxygène actif.

3. Cathéter à ballonnet selon l'une des revendications 1 à 2, dans lequel le rayon du ballonnet de cathéter est agrandi de plus de 15 % après déploiement complet, par augmentation de pression à l'intérieur du ballonnet de cathéter.

4. Cathéter à ballonnet selon l'une des revendications 1 à 3, dans lequel le ballonnet de cathéter a une pression d'éclatement inférieure à 10 000 hPa.

5. Cathéter à ballonnet selon l'une des revendications 1 à 4, dans lequel le ballonnet de cathéter possède, après déploiement complet, un diamètre supérieur de 20 % par rapport au diamètre de référence de l'artère.

6. Cathéter à ballonnet selon l'une des revendications 1 à 5, dans lequel la membrane de ballonnet du ballonnet de cathéter a été revêtue avec au moins une substance active hydrophile.

7. Cathéter à ballonnet selon l'une des revendications 1 à 6, dans lequel la membrane de ballonnet du ballonnet de cathéter est revêtue avec au moins une substance active appliquée de façon ouverte sur sa surface, de telle sorte que l'au moins une substance active soit libérée immédiatement lors de l'expansion du ballonnet de cathéter, dans lequel l'au moins une substance active en elle-même soluble dans l'eau se présente sous la forme d'un sel difficilement soluble dans l'eau ou d'un acide difficilement soluble dans l'eau ou d'une base difficilement soluble dans l'eau ou d'un composé complexe difficilement soluble dans l'eau.

8. Cathéter à ballonnet selon l'une des revendications 1 à 7, dans lequel la membrane de ballonnet du ballonnet de cathéter est revêtue avec au moins une substance active appliquée de façon ouverte sur sa surface, de telle sorte que l'au moins une substance active soit libérée immédiatement lors de l'expansion du ballonnet de cathéter, dans lequel l'au moins une substance active a été convertie après l'application sur la membrane de ballonnet en une forme difficilement soluble dans l'eau.

9. Cathéter à ballonnet selon l'une des revendications 1 à 8, dans lequel l'au moins une substance active ou l'au moins une substance active hydrophile est recouverte ou imbibée d'une couche biocompatible peu ou lentement soluble dans l'eau.

10. Cathéter à ballonnet selon l'une des revendications 1 à 9, dans lequel le ballonnet de cathéter contient un revêtement protecteur quelconque.

11. Cathéter à ballonnet selon l'une des revendications 1 à 10, dans lequel le ballonnet de cathéter a été revêtu avec une préparation qui contient au moins un solvant légèrement volatil ou au moins un composé chloré ou un composé fluoré présentant un point d'ébullition inférieur à 300 °C.

12. Cathéter à ballonnet selon l'une des revendications 1 à 11, dans lequel le ballonnet de cathéter est revêtu d'un gel dans lequel l'au moins une substance active fait office elle-même d'agent gélifiant ou participe à la gélification.

13. Cathéter à ballonnet selon l'une des revendications 1 à 12, dans lequel la composition de revêtement contient au moins un solvant hydrophile ou un mélange d'au moins un solvant hydrophile avec de l'eau.

14. Cathéter à ballonnet selon l'une des revendications 1 à 13, dans lequel la membrane de ballonnet du ballonnet de cathéter est revêtue avec une substance active dissoute dans un solvant organique qui contient au moins 1 % d'eau, séchée et stérilisée et dans lequel la substance active sur la membrane de ballonnet se présente sous forme cristalline.

15. Cathéter à ballonnet selon l'une des revendications 1 à 14, dans lequel le ballonnet de cathéter présentant une membrane de ballonnet à paroi lisse est revêtu avec une substance active dissoute dans un solvant organique qui contient au moins 1 % d'eau, séché et stérilisé et dans lequel la substance active se présente sous forme cristalline.

16. Cathéter à ballonnet selon la revendication 15, dans lequel le ballonnet de cathéter présentant une membrane de ballonnet à paroi lisse est revêtu à l'état replié.

17. Cathéter à ballonnet selon l'une des revendications 13 à 16, dans lequel le solvant organique contient du méthanol, de l'éthanol, du propanol, de l'isopropanol, de l'acétone, du tétrahydrofurane, de l'acide acétique, du dioxane ou du diméthylformamide.

18. Cathéter à ballonnet selon l'une des revendications 1 à 17, dans lequel la dose totale de tous les composants non volatils appliquée sur la membrane de ballonnet est inférieure à 10 µg/mm².

19. Cathéter à ballonnet selon l'une des revendications 1 à 18, contenant de l'acide méthotrexique, de l'arsenic ou des composés d'arsenic, du bismuth ou des composés de bismuth ou de la thalidomide.

20. Cathéter à ballonnet selon l'une des revendications 1 à 19, approprié pour le traitement local et la prophylaxie de maladies vasculaires et/ou pour le traitement de modifications vasculaires qui ne limitent pas substantiellement l'écoulement sanguin.

21. Procédé de production de ballonnets de cathéter d'un cathéter à ballonnet selon l'une des revendications 1 à 20, comprenant les étapes de :
a) mise à disposition d'un ballonnet de cathéter avec une membrane de ballonnet qui est hydrophile ou rendue hydrophile et/ou la surface de la membrane de ballonnet porte un revêtement hydrophile,
b) mise à disposition d'une unité de microdistribution contenant une composition de revêtement qui n'est pas en contact avec une phase gazeuse, dans lequel la composition de revêtement comprend une substance active et de l'urée,
c) revêtement sans perte et uniforme du ballonnet de cathéter avec la composition de revêtement en utilisant l'unité de microdistribution.

22. Procédé selon la revendication 21, dans lequel le ballonnet de cathéter tourne autour de son axe longitudinal pendant le revêtement et l'unité de microdistribution effectue un mouvement de va-et-vient le long de l'axe longitudinal du ballonnet de cathéter.
